(19) Europäisches Patentamt
European Patent Office
Office européen des brevets

(11) EP 3 587 469 A1

(12) **EUROPÄISCHE PATENTANMELDUNG**

(43) Veröffentlichungstag:
**01.01.2020 Patentblatt 2020/01**

(51) Int Cl.:
***C08G 64/34*** *(2006.01)* ***C08G 64/40*** *(2006.01)*
***C08G 65/26*** *(2006.01)* ***C08G 65/30*** *(2006.01)*

(21) Anmeldenummer: **18179395.1**

(22) Anmeldetag: **22.06.2018**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder: **Covestro Deutschland AG**
**51373 Leverkusen (DE)**

(72) Erfinder: **Die Erfindernennung liegt noch nicht vor**

(74) Vertreter: **Levpat**
**c/o Covestro AG**
**Gebäude 4825**
**51365 Leverkusen (DE)**

(54) **VERFAHREN ZUR HERSTELLUNG VON POLYOL**

(57) Gegenstand der Erfindung ist ein Verfahren zur Herstellung von Polyol, dadurch gekennzeichnet, dass in einer ersten Verfahrensstufe ein Diol hergestellt wird durch einen Prozess enthaltend die Schritte
(1-i) Anlagerung von Alkylenoxid und Kohlendioxid an eine H-funktionelle Startersubstanz in Anwesenheit eines Katalysators, unter Erhalt von Polyethercarbonatpolyol und cyclischen Carbonats,
(1-ii) Abtrennung des cyclischen Carbonats von der resultierenden Reaktionsmischung aus Schritt (1-i),
(1-iii) Hydrolytische Spaltung des aus Schritt (1-ii) abgetrennten cyclischen Carbonats zu Kohlendioxid und Diol,
(1-iv) gegebenenfalls destillative Reinigung des aus Schritt (1-iii) resultierenden Diols.

EP 3 587 469 A1

## Beschreibung

Die vorliegende Erfindung betrifft ein Verfahren zur Herstellung von Polyol durch die Anlagerung von Alkylenoxid und gegebenenfalls Kohlendioxid, cyclischen Carbonsäureanhydriden und/oder cyclischen Estern an Diol und gegebenenfalls weiterer H-funktioneller Startersubstanz, sowie durch Umsetzung von Carbonsäure, cyclisches Carbonsäureanhydrid, nicht-cyclische Ester und/oder cyclische Ester mit Diol und gegebenenfalls weiteren Alkoholen, wobei das Diol hergestellt ist aus cyclischem Carbonat, welches aus der Herstellung von Polyethercarbonatpolyolen erhalten wird.

Die Herstellung von Polyethercarbonatpolyolen durch katalytische Umsetzung von Alkylenoxiden (Epoxiden) und Kohlendioxid in Anwesenheit von H-funktionellen Startersubstanzen ("Starter") wird seit mehr als 40 Jahren intensiv untersucht (z. B. Inoue et al., Copolymerization of Carbon Dioxide and Epoxide with Organometallic Compounds; Die Makromolekulare Chemie 130, 210-220, 1969). Diese Reaktion ist in Schema (I) schematisch dargestellt, wobei R für einen organischen Rest wie Alkyl, Alkylaryl oder Aryl steht, der jeweils auch Heteroatome wie beispielsweise O, S, Si usw. enthalten kann, und wobei e, f und g für eine ganzzahlige Zahl stehen, und wobei das hier im Schema (I) gezeigte Produkt für das Polyethercarbonatpolyol lediglich so verstanden werden soll, dass sich Blöcke mit der gezeigten Struktur im erhaltenen Polyethercarbonatpolyol prinzipiell wiederfinden können, die Reihenfolge, Anzahl und Länge der Blöcke sowie die OH-Funktionalität des Starters aber variieren kann und nicht auf das in Schema (I) gezeigte Polyethercarbonatpolyol beschränkt ist. Diese Reaktion (siehe Schema (I)) ist ökologisch sehr vorteilhaft, da diese Reaktion die Umsetzung eines Treibhausgases wie $CO_2$ zu einem Polymer darstellt und so eine entsprechende Menge Alkylenoxid ersetzt, d.h. einspart. Als Nebenprodukt, entsteht das in Schema (I) gezeigte cyclische Carbonat (beispielsweise für R = $CH_3$ Propylencarbonat, im Folgenden auch als cPC bezeichnet, oder für R = H Ethylencarbonat, im Folgenden auch als cEC bezeichnet).

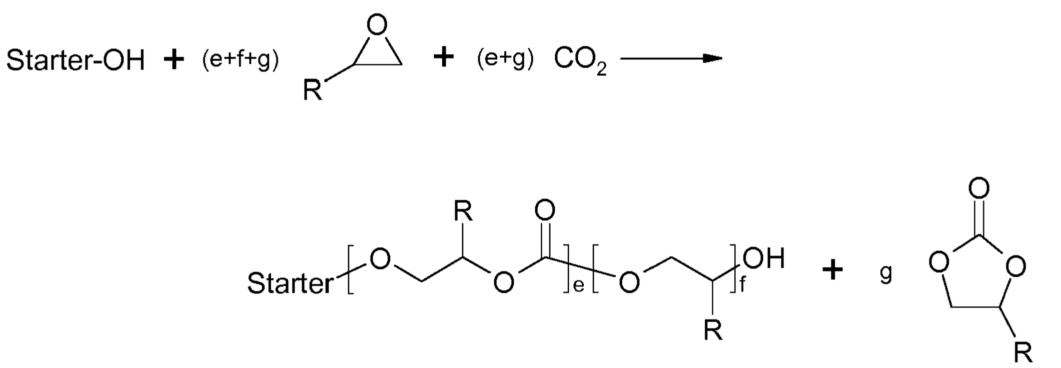

(I)

Für die Verwertung der als Nebenprodukte anfallenden cyclischen Carbonate wird zum Beispiel die Umsetzung der cyclischen Carbonate mit Aminen offenbart. Die erhaltenen Urethandiole (WO 2015/075057 A1 und WO 2015/091246 A1) bzw. Diurethandiole (WO 2016/188838 A1 und WO 2016/188992 A1) werden als H-funktionelle Startersubstanz zur Herstellung von Polyolen wie z.B. Polyetherpolyole oder Polycarbonatpolyole eingesetzt. Die Herstellung von Diolen aus cyclischen Carbonaten ist beispielsweise aus WO 2009/071651 A1 und US 6,080,897 bekannt. Die Verfahren offenbaren, dass durch Anlagerung von $CO_2$ an Alkylenoxid cyclisches Carbonat hergestellt und mit anschließender Hydrolyse in Anwesenheit von Wasser und eines Katalysators ein Diol erhalten werden kann. Die Herstellung des cyclischen Carbonats wird dabei bereits in Anwesenheit von Wasser durchgeführt. Nachteile dieser Verfahren sind unter anderem der energetische und technische Aufwand zur Herstellung des cyclischen Carbonats und die Verwendung von frischem Alkylenoxid.

Die der vorliegenden Erfindung zu Grunde liegende Aufgabe bestand deshalb darin, die Ausbeute bezüglich des bei der Herstellung von Polyethercarbonatpolyolen eingesetzten Alkylenoxids zu maximieren, d.h. das als Nebenprodukt anfallende cyclische Carbonat einer stofflichen Verwertung in einer Polyol-Anwendung zuzuführen.

Diese Aufgabe wurde erfindungsgemäß gelöst durch ein Verfahren zur Herstellung von Polyol, dadurch gekennzeichnet, dass in einer ersten Verfahrensstufe ein Diol hergestellt wird durch einen Prozess enthaltend die Schritte

(1-i) Anlagerung von Alkylenoxid und Kohlendioxid an eine H-funktionelle Startersubstanz in Anwesenheit eines Katalysators, unter Erhalt von Polyethercarbonatpolyol und cyclischen Carbonats,
(1-ii) Abtrennung des cyclischen Carbonats von der resultierenden Reaktionsmischung aus Schritt (1-i),
(1-iii) Hydrolytische Spaltung des aus Schritt (1-ii) abgetrennten cyclischen Carbonats zu Kohlendioxid und Diol,
(1-iv) gegebenenfalls destillative Reinigung des aus Schritt (1-iii) resultierenden Diols.

Vorzugsweise wird in einer zweiten Verfahrensstufe

(2-i) Polyol erhalten durch

a) Anlagerung von Alkylenoxid und gegebenenfalls Kohlendioxid, cyclisches Carbonsäureanhydrid und/oder cyclische Ester an das in der ersten Verfahrensstufe resultierende Diol und gegebenenfalls weiterer H-funktioneller Startersubstanz
oder
b) Umsetzung von Carbonsäure, cyclischem Carbonsäureanhydrid, nicht-cyclischem Ester und/oder cyclischem Ester mit dem in der ersten Verfahrensstufe resultierenden Diol und gegebenenfalls weiteren Alkoholen.

Überraschenderweise wurde gefunden, dass das bei der Anlagerung von Alkylenoxid und Kohlendioxid an eine H-funktionelle Startersubstanz gebildete cyclische Carbonat weiter umgesetzt werden kann zu einem Diol, welches für die Herstellung von Polyol eingesetzt werden kann. Dieses Verfahren führt nicht nur zu einer vollständigen Verwertung des für die Herstellung von Polyethercarbonatpolyolen eingesetzten Alkylenoxids, sondern führt durch den Einsatz des als Nebenprodukt in der Herstellung von Polyethercarbonatpolyolen gebildeten cyclischen Carbonats zu einem kostengünstigeren und nachhaltigeren Verfahren.

Schritt (1-i):

In Schritt (1-i) wird durch die Anlagerung von Alkylenoxid und Kohlendioxid an eine H-funktionelle Startersubstanz in Anwesenheit eines Katalysators Polyethercarbonatpolyol und cyclisches Carbonat erhalten, bevorzugt durch ein Verfahren dadurch gekennzeichnet, dass

(α) eine Teilmenge H-funktioneller Startersubstanz und/oder ein Suspensionsmittel, das keine H-funktionelle Gruppen aufweist, in einem Reaktor gegebenenfalls gemeinsam mit Katalysator vorgelegt wird,
(β) gegebenenfalls zur Aktivierung eines DMC-Katalysators eine Teilmenge (bezogen auf die Gesamtmenge der bei der Aktivierung und Copolymerisation eingesetzten Menge an Alkylenoxid) von Alkylenoxid zu der aus Schritt (α) resultierenden Mischung zugesetzt wird, wobei diese Zugabe einer Teilmenge an Alkylenoxid gegebenenfalls in Gegenwart von $CO_2$ erfolgen kann, und wobei dann die aufgrund der folgenden exothermen chemischen Reaktion auftretende Temperaturspitze ("Hotspot") und/oder ein Druckabfall im Reaktor jeweils abgewartet wird, und wobei der Schritt (β) zur Aktivierung auch mehrfach erfolgen kann,
(γ) eine H-funktionelle Startersubstanz, Alkylenoxid und gegebenenfalls ein Suspensionsmittel, das keine H-funktionelle Gruppen aufweist, und/oder Kohlendioxid während der Reaktion in den Reaktor zudosiert werden,
(δ) gegebenenfalls das in Schritt (γ) kontinuierlich entfernte Reaktionsgemisch in einen Nachreaktor überführt wird, in dem im Wege einer Nachreaktion der Gehalt an freiem Alkylenoxid im Reaktionsgemisch reduziert wird.

Als Katalysator wird in Schritt (1-i) mindestens eine Verbindung eingesetzt, welche die Anlagerung von Alkylenoxid und Kohlendioxid katalysiert, bevorzugt mindestens ein Doppelmetallcyanid-Katalysator oder mindestens ein Metallkomplexkatalysator auf Basis der Metalle Zink und/oder Cobalt, besonders bevorzugt ein Doppelmetallcyanid-Katalysator.

Zu (α):

Die in (α) eingesetzte Teilmenge der H-funktionellen Startersubstanz kann Komponente K enthalten, bevorzugt in einer Menge von mindestens 100 ppm, besonders bevorzugt von 100 bis 10000 ppm.

Bei dem Verfahren kann zunächst eine Teilmenge H-funktioneller Startersubstanz und/oder ein Suspensionsmittel, das keine H-funktionelle Gruppen aufweist, im Reaktor vorgelegt werden. Anschließend wird gegebenenfalls die für die Polyaddition benötigte Menge an Katalysator in den Reaktor gegeben. Die Reihenfolge der Zugabe ist dabei nicht entscheidend. Es kann auch erst der Katalysator und anschließend eine Teilmenge H-funktioneller Startersubstanz in den Reaktor gefüllt werden. Alternativ kann auch erst der Katalysator in einer Teilmenge H-funktioneller Startersubstanz suspendiert und anschließend die Suspension in den Reaktor gefüllt werden.

In einer bevorzugten Ausführungsform der Erfindung wird in Schritt (α) eine H-funktionelle Startersubstanz im Reaktor gegebenenfalls gemeinsam mit Katalysator vorgelegt, und dabei wird kein Suspensionsmittel, das keine H-funktionelle Gruppen enthält, im Reaktor vorgelegt.

Der Katalysator wird vorzugsweise in einer Menge eingesetzt, so dass der Gehalt an Katalysator im resultierenden Reaktionsprodukt 10 bis 10000 ppm, besonders bevorzugt 20 bis 5000 ppm und höchst bevorzugt 50 bis 500 ppm beträgt

In einer bevorzugten Ausführungsform wird in das resultierende Gemisch aus (a) einer Teilmenge H-funktioneller Startersubstanz und (b) Katalysator bei einer Temperatur von 90 bis 150 °C, besonders bevorzugt von 100 bis

140 °C Inertgas (beispielsweise Argon oder Stickstoff), ein Inertgas-Kohlendioxid-Gemisch oder Kohlendioxid eingeleitet und gleichzeitig ein reduzierter Druck (absolut) von 10 mbar bis 800 mbar, besonders bevorzugt von 50 mbar bis 200 mbar angelegt.

In einer alternativen bevorzugten Ausführungsform wird das resultierende Gemisch aus (a) einer Teilmenge H-funktioneller Startersubstanz und (b) Katalysator bei einer Temperatur von 90 bis 150 °C, besonders bevorzugt von 100 bis 140 °C mindestens einmal, vorzugsweise dreimal mit 1,5 bar bis 10 bar (absolut), besonders bevorzugt 3 bar bis 6 bar (absolut) eines Inertgases (beispielsweise Argon oder Stickstoff), eines Inertgas-Kohlendioxid-Gemisches oder Kohlendioxid beaufschlagt und jeweils anschließend der Überdruck auf ca. 1 bar (absolut) reduziert.

Der Katalysator kann in fester Form oder als Suspension in Suspensionsmittel, das keine H-funktionelle Gruppen enthält, in H-funktioneller Startersubstanz oder in einer Mischung aus den genannten zugegeben werden.

In einer weiteren bevorzugten Ausführungsform wird in Schritt (α)

(α-I) eine Teilmenge H-funktioneller Startersubstanz vorgelegt und

(α-II) die Temperatur der Teilmenge H-funktioneller Startersubstanz auf 50 bis 200 °C, bevorzugt 80 bis 160 °C, besonders bevorzugt 100 bis 140 °C gebracht und/oder der Druck im Reaktor auf weniger als 500 mbar, bevorzugt 5 mbar bis 100 mbar erniedrigt, wobei gegebenenfalls ein Inertgas-Strom (beispielsweise von Argon oder Stickstoff), ein Inertgas-Kohlendioxid-Strom oder ein Kohlendioxid-Strom durch den Reaktor geleitet wird,

wobei der Katalysator zur Teilmenge H-funktioneller Startersubstanz in Schritt (α-I) oder unmittelbar anschließend in Schritt (α-II) zugesetzt wird.

<u>Zu Schritt (β):</u>

Schritt (β) dient der Aktivierung des DMC-Katalysators. Dieser Schritt kann gegebenenfalls unter Inertgasatmosphäre, unter einer Atmosphäre aus Inertgas-Kohlendioxid-Gemisch oder unter einer Kohlendioxid-Atmosphäre durchgeführt werden. Als Aktivierung im Sinne dieser Erfindung wird ein Schritt bezeichnet, bei dem eine Teilmenge Alkylenoxid bei Temperaturen von 90 bis 150 °C zur DMC-KatalysatorSuspension zugegeben wird und dann die Zugabe des Alkylenoxids unterbrochen wird, wobei aufgrund einer folgenden exothermen chemischen Reaktion eine Wärmeentwicklung, die zu einer Temperaturspitze ("Hotspot") führen kann, sowie aufgrund der Umsetzung von Alkylenoxid und gegebenenfalls $CO_2$ ein Druckabfall im Reaktor beobachtet wird. Der Verfahrensschritt der Aktivierung ist die Zeitspanne von der Zugabe der Teilmenge an Alkylenoxid, gegebenenfalls in Gegenwart von $CO_2$, zum DMC-Katalysator bis zum Auftreten der Wärmeentwicklung. Gegebenenfalls kann die Teilmenge des Alkylenoxids in mehreren Einzelschritten, gegebenenfalls in Gegenwart von $CO_2$, zum DMC-Katalysator gegeben werden und dann jeweils die Zugabe des Alkylenoxids unterbrochen werden. In diesem Fall umfasst der Verfahrensschritt der Aktivierung die Zeitspanne von der Zugabe der ersten Teilmenge an Alkylenoxid, gegebenenfalls in Gegenwart von $CO_2$, zum DMC-Katalysator bis zum Auftreten der Wärmeentwicklung nach Zugabe der letzten Teilmenge an Alkylenoxid. Im Allgemeinen kann dem Aktivierungsschritt ein Schritt zum Trocknen des DMC-Katalysators und ggf. der H-funktionellen Startersubstanz bei erhöhter Temperatur und/oder reduziertem Druck, gegebenenfalls unter Durchleiten eines Inertgases durch die Reaktionsmischung, vorgelagert sein.

Die Dosierung des Alkylenoxids (und gegebenenfalls des Kohlendioxids) kann prinzipiell in unterschiedlicher Weise erfolgen. Der Start der Dosierung kann aus dem Vakuum heraus oder bei einem zuvor gewählten Vordruck erfolgen. Der Vordruck wird bevorzugt durch Einleiten eines Inertgases (wie beispielsweise Stickstoff oder Argon) oder von Kohlendioxid eingestellt, wobei der Druck(absolut) 5 mbar bis 100 bar, vorzugsweise 10 mbar bis 50 bar und bevorzugt 20 mbar bis 50 bar beträgt.

In einer bevorzugten Ausführungsform beträgt die bei der Aktivierung in Schritt (β) eingesetzte Menge des Alkylenoxids 0,1 bis 25,0 Gew.-%, bevorzugt 1,0 bis 20,0 Gew.-%, besonders bevorzugt 2,0 bis 16,0 Gew.-% (bezogen auf die im Schritt (α) eingesetzte Menge an H-funktioneller Startersubstanz). Das Alkylenoxid kann in einem Schritt oder portionsweise in mehreren Teilmengen zugegeben werden. Bevorzugt wird nach Zugabe einer Teilmenge des Alkylenoxids die Zugabe des Alkylenoxids bis zum Auftreten der Wärmeentwicklung unterbrochen und erst dann die nächste Teilmenge an Alkylenoxid zugegeben. Bevorzugt ist auch eine zweistufige Aktivierung (Schritt β), wobei

(β1) in einer ersten Aktivierungsstufe die Zugabe einer ersten Teilmenge von Alkylenoxid unter Inertgasatmosphäre erfolgt und

(β2) in einer zweiten Aktivierungsstufe die Zugabe einer zweiten Teilmenge von Alkylenoxid unter Kohlendioxid-Atmosphäre erfolgt.

Zu Schritt (γ):

Die Dosierung H-funktioneller Startersubstanz, Alkylenoxids und gegebenenfalls eines Suspensionsmittels, das keine H-funktionelle Gruppen aufweist, und/oder von Kohlendioxid kann simultan oder sequentiell (portionsweise) erfolgen, beispielsweise kann die gesamte Kohlendioxidmenge, die Menge an H-funktioneller Startersubstanz, des Suspensionsmittels, das keine H-funktionelle Gruppen aufweist, und/oder die in Schritt (γ) dosierte Menge an Alkylenoxid auf einmal oder kontinuierlich zugegeben werden. Der hier verwendete Begriff "kontinuierlich" kann als Modus der Zugabe eines Reaktanden so definiert werden, dass eine für die Copolymerisation wirksame Konzentration des Reaktanden aufrechterhalten wird, d.h. beispielsweise kann die Dosierung mit einer konstanten Dosierrate, mit einer variierenden Dosierrate oder portionsweise erfolgen.

Es ist möglich, während der Zugabe des Alkylenoxids, des Suspensionsmittels, das keine H-funktionelle Gruppen aufweist, und/oder H-funktioneller Startersubstanz den $CO_2$-Druck allmählich oder schrittweise zu steigern oder zu senken oder gleich zu lassen. Vorzugsweise wird der Gesamtdruck während der Reaktion durch eine Nachdosierung an Kohlendioxid konstant gehalten. Die Dosierung des Alkylenoxids, des Suspensionsmittels, das keine H-funktionelle Gruppen aufweist, und/oder H-funktioneller Startersubstanz erfolgt simultan oder sequentiell zur Kohlendioxid-Dosierung. Es ist möglich, das Alkylenoxid mit konstanter Dosierrate zu dosieren oder die Dosierrate allmählich oder schrittweise zu steigern oder zu senken oder das Alkylenoxid portionsweise zuzugeben. Bevorzugt wird das Alkylenoxid mit konstanter Dosierrate dem Reaktionsgemisch zugegeben. Werden mehrere Alkylenoxide zur Synthese der Polyethercarbonatpolyole eingesetzt, so können die Alkylenoxide einzeln oder als Gemisch dosiert werden. Die Dosierung des Alkylenoxids, des Suspensionsmittels, das keine H-funktionelle Gruppen aufweist, bzw. der H-funktionellen Startersubstanz kann simultan oder sequentiell über jeweils separate Dosierungen (Zugaben) erfolgen oder über eine oder mehrere Dosierungen, wobei Alkylenoxid, das Suspensionsmittel, das keine H-funktionelle Gruppen aufweist, bzw. die H-funktionelle Startersubstanz einzeln oder als Gemisch dosiert werden können. Über die Art und/oder Reihenfolge der Dosierung der H-funktionellen Startersubstanz, des Alkylenoxids, des Suspensionsmittels, das keine H-funktionelle Gruppen aufweist, und/oder des Kohlendioxids ist es möglich, statistische, alternierende, blockartige oder gradientenartige Polyethercarbonatpolyole zu synthetisieren.

Vorzugsweise wird ein Überschuss an Kohlendioxid bezogen auf die berechnete Menge an einzubauendem Kohlendioxid im Polyethercarbonatpolyol eingesetzt, da bedingt durch die Reaktionsträgheit von Kohlendioxid ein Überschuss von Kohlendioxid von Vorteil ist. Die Menge an Kohlendioxid kann über den Gesamtdruck bei den jeweiligen Reaktionsbedingungen festgelegt werden. Als Gesamtdruck (absolut) hat sich der Bereich von 0,01 bis 120 bar, bevorzugt 0,1 bis 110 bar, besonders bevorzugt von 1 bis 100 bar für die Copolymerisation zur Herstellung der Polyethercarbonatpolyole als vorteilhaft erwiesen. Es ist möglich, das Kohlendioxid kontinuierlich oder diskontinuierlich zuzuführen. Dies hängt davon ab, wie schnell das Alkylenoxid verbraucht wird und ob das Produkt gegebenenfalls CO2-freie Polyether-Blöcke enthalten soll. Die Menge des Kohlendioxids (angegeben als Druck) kann bei der Zugabe des Alkylenoxids ebenso variieren. $CO_2$ kann auch als Feststoff in den Reaktor gegeben werden und dann unter den gewählten Reaktionsbedingungen in den gasförmigen, gelösten, flüssigen und/oder überkritischen Zustand übergehen.

Für das erfindungsgemäße Verfahren hat sich weiterhin gezeigt, dass die Copolymerisation (Schritt (γ)) zur Herstellung der Polyethercarbonatpolyole vorteilhafterweise bei 50 bis 150 °C, bevorzugt bei 60 bis 145 °C, besonders bevorzugt bei 70 bis 140 °C und ganz besonders bevorzugt bei 90 bis 130 °C durchgeführt wird. Werden Temperaturen unterhalb von 50 °C eingestellt, wird die Reaktion im Allgemeinen sehr langsam. Bei Temperaturen oberhalb von 150 °C steigt die Menge an unerwünschten Nebenprodukten stark an. Gegebenenfalls kann in Schritt (γ) ebenfalls Katalysator zudosiert werden. Die Dosierung des Alkylenoxids, H-funktioneller Startersubstanz, des Suspensionsmittels, das keine H-funktionelle Gruppen aufweist, und des Katalysators kann über separate oder gemeinsame Dosierstellen erfolgen. In einer bevorzugten Ausführungsform werden Alkylenoxid, H-funktionelle Startersubstanz und gegebenenfalls Suspensionsmittel, das keine H-funktionelle Gruppen aufweist, über separate Dosierstellen kontinuierlich dem Reaktionsgemisch zugeführt. Diese Zugabe H-funktioneller Startersubstanz und des Suspensionsmittels, das keine H-funktionelle Gruppen aufweist, kann als kontinuierliche Zudosierung in den Reaktor oder portionsweise erfolgen.

Die Schritte (α), (β) und (γ) können in demselben Reaktor oder jeweils separat in unterschiedlichen Reaktoren durchgeführt werden. Besonders bevorzugte Reaktortypen sind: Rohrreaktoren, Rührkessel, Schlaufenreaktoren.

Polyethercarbonatpolyole können in einem Rührkessel hergestellt werden, wobei der Rührkessel je nach Ausführungsform und Betriebsweise über den Reaktormantel, innen liegende und/oder in einem Umpumpkreislauf befindliche Kühlflächen gekühlt wird. Sowohl in der semi-batch Anwendung, bei der das Produkt erst nach Ende der Reaktion entnommen wird, als auch in der kontinuierlichen Anwendung, bei der das Produkt kontinuierlich entnommen wird, ist besonders auf die Dosiergeschwindigkeit des Alkylenoxids zu achten. Sie ist so einzustellen, dass trotz der inhibierenden Wirkung des Kohlendioxids das Alkylenoxid genügend schnell abreagiert. Die Konzentration an freien Alkylenoxid in der Reaktionsmischung während des Aktivierungsschritts (Schritt β) beträgt vorzugsweise > 0 bis 100 Gew.-%, besonders bevorzugt > 0 bis 50 Gew-%, höchst bevorzugt > 0 bis 20 Gew.-% (jeweils bezogen auf das Gewicht der Reaktionsmischung). Die Konzentration an freien Alkylenoxid in der Reaktionsmischung während der Reaktion (Schritt γ) beträgt

vorzugsweise > 0 bis 40 Gew.-%, besonders bevorzugt > 0 bis 25 Gew.-%, höchst bevorzugt > 0 bis 15 Gew.-% (jeweils bezogen auf das Gewicht der Reaktionsmischung).

In einer bevorzugten Ausführungsform wird die gemäß den Schritten (α) und (β) resultierende aktivierten DMC-Katalysator enthaltende Mischung in demselben Reaktor weiter mit Alkylenoxid, H-funktioneller Startersubstanz, gegebenenfalls Suspensionsmittel, das keine H-funktionelle Gruppen aufweist, und Kohlendioxid umgesetzt. In einer weiteren bevorzugten Ausführungsform wird die gemäß den Schritten (α) und (β) resultierende aktivierte DMC-Katalysator enthaltende Mischung in einem anderen Reaktionsbehältnis (beispielsweise einem Rührkessel, Rohrreaktor oder Schlaufenreaktor) weiter mit Alkylenoxid, H-funktioneller Startersubstanz, gegebenenfalls Suspensionsmittel, das keine H-funktionelle Gruppen aufweist, und Kohlendioxid umgesetzt.

Bei Reaktionsführung in einem Rohrreaktor werden die gemäß den Schritten (α) und (β) resultierende aktivierte DMC-Katalysator enthaltende Mischung, H-funktionelle Startersubstanz, Alkylenoxid, gegebenenfalls Suspensionsmittel, das keine H-funktionelle Gruppen aufweist, und Kohlendioxid kontinuierlich durch ein Rohr gepumpt. Die molaren Verhältnisse der Reaktionspartner variieren je nach gewünschtem Polymer. In einer bevorzugten Ausführungsform wird hierbei Kohlendioxid in seiner flüssigen oder überkritischen Form zudosiert, um eine optimale Mischbarkeit der Komponenten zu ermöglichen. Vorteilhafterweise werden Mischelemente zur besseren Durchmischung der Reaktionspartner eingebaut, wie sie zum Beispiel von der Firma Ehrfeld Mikrotechnik BTS GmbH vertrieben werden, oder Mischer-Wärmetauscherelemente, die gleichzeitig die Durchmischung und Wärmeabfuhr verbessern.

Schlaufenreaktoren können ebenfalls zur Herstellung von Polyethercarbonatpolyolen verwendet werden. Hierunter fallen im Allgemeinen Reaktoren mit Stoffrückführung, wie beispielsweise ein Strahlschlaufenreaktor, der auch kontinuierlich betrieben werden kann, oder ein schlaufenförmig ausgelegter Rohrreaktor mit geeigneten Vorrichtungen für die Umwälzung der Reaktionsmischung oder eine Schlaufe von mehreren hintereinander geschalteten Rohrreaktoren. Der Einsatz eines Schlaufenreaktors ist insbesondere deshalb von Vorteil, weil hier eine Rückvermischung realisiert werden kann, so dass die Konzentration an freien Alkylenoxid in der Reaktionsmischung im optimalen Bereich, vorzugsweise im Bereich > 0 bis 40 Gew.-%, besonders bevorzugt > 0 bis 25 Gew.-%, höchst bevorzugt > 0 bis 15 Gew.-% (jeweils bezogen auf das Gewicht der Reaktionsmischung) gehalten werden kann.

Bevorzugt werden die Polyethercarbonatpolyole in einem kontinuierlichen Verfahren hergestellt, das sowohl eine kontinuierliche Copolymerisation als auch eine kontinuierliche Zugabe H-funktioneller Startersubstanz und gegebenenfalls Suspensionsmittels, das keine H-funktionelle Gruppen aufweist, umfasst.

Gegenstand der Erfindung ist daher auch ein Verfahren, wobei in Schritt (γ) H-funktionelle Startersubstanz, Alkylenoxid, gegebenenfalls Suspensionsmittel, das keine H-funktionelle Gruppen aufweist, sowie DMC-Katalysator in Gegenwart von Kohlendioxid ("Copolymerisation") kontinuierlich in den Reaktor dosiert werden und wobei das resultierende Reaktionsgemisch (enthaltend Polyethercarbonatpolyol und cyclisches Carbonat) kontinuierlich aus dem Reaktor entfernt wird. Vorzugsweise wird dabei in Schritt (γ) der DMC-Katalysator in H-funktioneller Startersubstanz suspendiert kontinuierlich zugegeben.

Beispielsweise wird für das kontinuierliche Verfahren zur Herstellung der Polyethercarbonatpolyole eine DMC-Katalysator-enthaltende Mischung hergestellt, dann werden gemäß Schritt (γ)

(γ1) jeweils eine Teilmenge H-funktioneller Startersubstanz, Alkylenoxid und Kohlendioxid zudosiert zum Initiieren der Copolymerisation, und

(γ2) während des Fortschreitens der Copolymerisation jeweils die verbleibende Menge an DMC-Katalysator, H-funktioneller Startersubstanz, gegebenenfalls Suspensionsmittel, das keine H-funktionelle Gruppen aufweist, und Alkylenoxid in Gegenwart von Kohlendioxid kontinuierlich zudosiert, wobei gleichzeitig resultierendes Reaktionsgemisch kontinuierlich aus dem Reaktor entfernt wird.

In Schritt (γ) wird der Katalysator bevorzugt suspendiert in H-funktioneller Startersubstanz zugegeben, wobei die Menge vorzugsweise so gewählt ist, dass der Gehalt an Katalysator im resultierenden Reaktionsprodukt 10 bis 10000 ppm, besonders bevorzugt 20 bis 5000 ppm und höchst bevorzugt 50 bis 500 ppm beträgt.

Vorzugsweise werden die Schritte (α) und (β) in einem ersten Reaktor durchgeführt, und das resultierende Reaktionsgemisch dann in einen zweiten Reaktor für die Copolymerisation gemäß Schritt (γ) überführt. Es ist aber auch möglich, die Schritte (α), (β) und (γ) in einem Reaktor durchzuführen.

Der hier verwendete Begriff "kontinuierlich" kann als Modus der Zugabe eines relevanten Katalysators oder Reaktanden so definiert werden, dass eine im Wesentlichen kontinuierlich wirksame Konzentration des Katalysators oder des Reaktanden aufrechterhalten wird. Die Katalysatorzufuhr kann echt kontinuierlich oder in relativ eng beabstandeten Inkrementen erfolgen. Gleichermaßen können eine kontinuierliche Zugabe H-funktioneller Startersubstanz und eine kontinuierliche Zugabe des Suspensionsmittels, das keine H-funktionellen Gruppen aufweist, echt kontinuierlich sein oder in Inkrementen erfolgen. Es würde nicht vom vorliegenden Verfahren abweichen, einen Katalysator oder Reaktanden inkrementell so zuzugeben, dass die Konzentration der zugegebenen Materialien für einige Zeit vor der nächsten inkrementellen Zugabe im Wesentlichen auf null abfällt. Es ist jedoch bevorzugt, dass die Katalysatorkonzentration während des Hauptteils des Verlaufs der kontinuierlichen Reaktion im Wesentlichen auf derselben Konzentration gehalten wird und dass H-funktionelle Startersubstanz während des Hauptteils des Copolymerisations-Verfahrens vorhanden ist. Eine inkrementelle Zugabe von Katalysator und/oder Reaktant, die die Beschaffenheit des Produkts nicht wesentlich beeinflusst, ist in demjenigen Sinn, in dem der Begriff hier verwendet wird, dennoch "kontinuierlich". Es ist beispielsweise machbar, eine Rückführungsschleife bereitzustellen, in der ein Teil der reagierenden Mischung zu einem vorherigen Punkt im Verfahren zurückgeführt wird, wodurch durch inkrementelle Zugaben bewirkte Diskontinuitäten geglättet werden.

Schritt (δ)

Gegebenenfalls kann in einem Schritt (δ) das Reaktionsgemisch in Schritt (γ) in einen Nachreaktor überführt werden, in dem im Wege einer Nachreaktion der Gehalt an freiem Alkylenoxid im Reaktionsgemisch reduziert wird. Als Nachreaktor kann beispielsweise ein Rohrreaktor, ein Schlaufenreaktor oder ein Rührkessel dienen.

Bevorzugt liegt der Druck in diesem Nachreaktor bei demselben Druck wie in dem Reaktionsapparat, in dem der Reaktionsschritt (γ) durchgeführt wird. Der Druck in dem nachgeschalteten Reaktor kann jedoch auch höher oder niedriger gewählt werden. In einer weiteren bevorzugten Ausführungsform wird das Kohlendioxid nach dem Reaktionsschritt (γ) ganz oder teilweise abgelassen und der nachgeschaltete Reaktor bei Normaldruck oder einem geringen Überdruck betrieben. Die Temperatur in dem nachgeschalteten Reaktor liegt bevorzugt bei 50 bis 150 °C und besonders bevorzugt bei 80 bis 140 °C.

Die erhaltenen Polyethercarbonatpolyole haben beispielsweise eine Funktionalität von mindestens 1, bevorzugt von 1 bis 8, besonders bevorzugt von 1 bis 6 und ganz besonders bevorzugt von 2 bis 4. Das Molekulargewicht beträgt bevorzugt 400 bis 10000 g/mol und besonders bevorzugt 500 bis 6000 g/mol.

Alkylenoxid

Allgemein können für das Verfahren Alkylenoxide (Epoxide) mit 2-24 Kohlenstoffatomen eingesetzt werden. Bei den Alkylenoxiden mit 2-24 Kohlenstoffatomen handelt es sich beispielsweise um eine oder mehrere Verbindungen ausgewählt aus der Gruppe bestehend aus Ethylenoxid, Propylenoxid, 1-Butenoxid, 2,3-Butenoxid, 2-Methyl-1,2-propenoxid (Isobutenoxid), 1-Pentenoxid, 2,3-Pentenoxid, 2-Methyl-1,2-butenoxid, 3-Methyl-1,2-butenoxid, 1-Hexenoxid, 2,3-Hexenoxid, 3,4-Hexenoxid, 2-Methyl-1,2-pentenoxid, 4-Methyl-1,2-pentenoxid, 2-Ethyl-1,2-butenoxid, 1-Heptenoxid, 1-Octenoxid, 1-Nonenoxid, 1-Decenoxid, 1-Undecenoxid, 1-Dodecenoxid, 4-Methyl-1,2-pentenoxid, Butadienmonoxid, Isoprenmonoxid, Cyclopentenoxid, Cyclohexenoxid, Cycloheptenoxid, Cyclooctenoxid, Styroloxid, Methylstyroloxid, Pinenoxid, ein- oder mehrfach epoxidierte Fette als Mono-, Di- und Triglyceride, epoxidierte Fettsäuren, $C_1$-$C_{24}$-Ester von epoxidierten Fettsäuren, Epichlorhydrin, Glycidol, und Derivate des Glycidols wie beispielsweise Methylglycidylether, Ethylglycidylether, 2-Ethylhexylglycidylether, Allylglycidylether, Glycidylmethacrylat sowie epoxidfunktionelle Alkyoxysilane wie beispielsweise 3-Glycidyloxypropyltrimethoxysilan, 3-Glycidyloxypropyltriethoxysilan, 3-Glycidyloxypropyltripropoxysilan, 3-Glycidyloxypropyl-methyl-dimethoxysilan, 3-Glycidyloxypropyl-ethyldiethoxysilan, 3-Glycidyloxypropyltriisopropoxysilan. Vorzugsweise werden als Alkylenoxid Ethylenoxid und/oder Propylenoxid, insbesondere Propylenoxid eingesetzt. In dem erfindungsgemäßen Verfahren kann als Alkylenoxid auch eine Mischung von Alkylenoxiden eingesetzt werden.

H-funktionelle Startersubstanz

Als geeignete H-funktionelle Startersubstanz ("Starter") können Verbindungen mit für die Alkoxylierung aktiven H-Atomen eingesetzt werden, die eine Molmasse von 18 bis 4500 g/mol, bevorzugt von 62 bis 500 g/mol und besonders bevorzugt von 62 bis 182 g/mol aufweisen.

Für die Alkoxylierung aktive Gruppen mit aktiven H-Atomen sind beispielsweise -OH, - $NH_2$ (primäre Amine), -NH- (sekundäre Amine), -SH und -$CO_2H$, bevorzugt sind -OH und -$NH_2$, besonders bevorzugt ist -OH. Als H-funktionelle Startersubstanz wird beispielsweise eine oder mehrere Verbindungen ausgewählt aus der Gruppe bestehend aus ein- oder mehrwertigen Alkoholen, mehrwertigen Aminen, mehrwertigen Thiolen, Aminoalkohole, Thioalkohole, Hydroxyester, Polyetherpolyole, Polyesterpolyole, Polyesteretherpolyole, Polyethercarbonatpolyole, Polycarbonatpolyole, Polycarbonate, Polyethylenimine, Polyetheramine, Polytetrahydrofurane (z. B. PolyTHF® der BASF), Polytetrahydrofuranamine, Polyetherthiole, Polyacrylatpolyole, Ricinusöl, das Mono- oder Diglycerid von Ricinolsäure, Monoglyceride von Fettsäuren, chemisch modifizierte Mono-, Di- und/oder Triglyceride von Fettsäuren, und $C_1$-$C_{24}$ Alkyl-Fettsäureester, die im Mittel mindestens 2 OH-Gruppen pro Molekül enthalten, eingesetzt. Beispielhaft handelt es sich bei den $C_1$-$C_{24}$ Alkyl-Fettsäureester, die im Mittel mindestens 2 OH-Gruppen pro Molekül enthalten, um Handelsprodukte wie Lupranol Balance® (Fa. BASF AG), Merginol®-Typen (Fa. Hobum Oleochemicals GmbH), Sovermol®-Typen (Fa. Cognis Deutschland GmbH & Co. KG) und Soyol®TM-Typen (Fa. USSC Co.).

Als monofunktionelle Startersubstanz können Alkohole, Amine, Thiole und Carbonsäuren eingesetzt werden. Als monofunktionelle Alkohole können Verwendung finden: Methanol, Ethanol, 1-Propanol, 2-Propanol, 1-Butanol, 2-Butanol, tert.-Butanol, 3-Buten-1-ol, 3-Butin-1-ol, 2-Methyl-3-buten-2-ol, 2-Methyl-3-butin-2-ol, Propagylalkohol, 2-Methyl-2-propanol, 1-tert-Butoxy-2-propanol, 1-Pentanol, 2-Pentanol, 3-Pentanol, 1-Hexanol, 2-Hexanol, 3-Hexanol, 1-Heptanol, 2-Heptanol, 3-Heptanol, 1-Octanol, 2-Octanol, 3-Octanol, 4-Octanol, Phenol, 2-Hydroxybiphenyl, 3-Hydroxybiphenyl, 4-Hydroxybiphenyl, 2-Hydroxypyridin, 3-Hydroxypyridin, 4-Hydroxypyridin. Als mono funktionelle Amine kommen in Frage: Butylamin, tert-Butylamin, Pentylamin, Hexylamin, Anilin, Aziridin, Pyrrolidin, Piperidin, Morpholin. Als monofunktionelle Thiole können verwendet werden: Ethanthiol, 1-Propanthiol, 2-Propanthiol, 1-Butanthiol, 3-Methyl-1-butanthiol, 2-Buten-1-thiol, Thiophenol. Als mono funktionelle Carbonsäuren seien genannt: Ameisensäure, Essigsäure, Propionsäure, Buttersäure, Fettsäuren wie Stearinsäure, Palmitinsäure, Ölsäure, Linolsäure, Linolensäure, Benzoesäure, Acrylsäure.

Als H-funktionelle Startersubstanz geeignete mehrwertige Alkohole sind beispielsweise zweiwertige Alkohole (wie beispielsweise Ethylenglykol, Diethylenglykol, Propylenglykol, Dipropylenglykol, 1,3-Propandiol, 2-Methyl-1,3-propandiol, 1,4-Butandiol, 1,4-Butendiol, 1,4-Butindiol, Neopentylglykol, 1,5-Pentandiol, Methylpentandiole (wie beispielweise 3-Methyl-1,5-pentandiol), 1,6-Hexandiol, 1,8-Octandiol, 1,10-Decandiol, 1,12-Dodecandiol, Bis-(hydroxymethyl)-cyclohexane (wie beispielweise 1,4-Bis-(hydroxymethyl)cyclohexan), Triethylenglykol, Tetraethylenglykol, Polyethylenglykole, Dipropylenglykol, Tripropylenglykol, Polypropylenglykole, Dibutylenglykol und Polybutylenglykole); dreiwertige Alkohole (wie beispielsweise Trimethylolpropan, Glycerin, Trishydroxyethylisocyanurat, Rizinusöl); vierwertige Alkohole (wie beispielsweise Pentaerythrit); Polyalkohole (wie beispielsweise Sorbit, Hexit, Saccharose, Stärke, Stärkehydrolysate, Cellulose, Cellulosehydrolysate, hydroxyfunktionalisierte Fette und Öle, insbesondere Rizinusöl), sowie alle Modifizierungsprodukte dieser zuvorgenannten Alkohole mit unterschiedlichen Mengen an ε-Caprolacton.

Die H-funktionelle Startersubstanz kann auch aus der Substanzklasse der Polyetherpolyole ausgewählt sein, die ein Molekulargewicht $M_n$ im Bereich von 18 bis 4500 g/mol und eine Funktionalität von 2 bis 3 aufweisen. Bevorzugt sind Polyetherpolyole, die aus sich wiederholenden Ethylenoxid- und Propylenoxideinheiten aufgebaut sind, bevorzugt mit einem Anteil von 35 bis 100% Propylenoxideinheiten, besonders bevorzugt mit einem Anteil von 50 bis 100% Propylenoxideinheiten. Hierbei kann es sich um statistische Copolymere, Gradienten-Copolymere, alternierende oder Blockcopolymere aus Ethylenoxid und Propylenoxid handeln.

Die H-funktionelle Startersubstanz kann auch aus der Substanzklasse der Polyesterpolyole ausgewählt sein. Als Polyesterpolyole werden mindestens difunktionelle Polyester eingesetzt. Bevorzugt bestehen Polyesterpolyole aus alternierenden Säure- und Alkoholeinheiten. Als Säurekomponenten werden z. B. Bernsteinsäure, Maleinsäure, Maleinsäureanhydrid, Adipinsäure, Phthalsäureanhydrid, Phthalsäure, Isophthalsäure, Terephthalsäure, Tetrahydrophtalsäure, Tetrahydrophthalsäureanhydrid, Hexahydrophthalsäureanhydrid oder Gemische aus den genannten Säuren und/oder Anhydriden eingesetzt. Als Alkoholkomponenten werden z. B. Ethandiol, 1,2-Propandiol, 1,3-Propandiol, 2-Methyl-1,3-propandiol, 1,4-Butandiol, 1,5-Pentandiol, Neopentylglykol, 1,6-Hexandiol, 1,4-Bis-(hydroxymethyl)-cyclohexan, Diethylenglykol, Dipropylenglykol, Trimethylolpropan, Glycerin, Pentaerythrit oder Gemische aus den genannten Alkoholen verwendet. Werden als Alkoholkomponente zweiwertige oder mehrwertige Polyetherpolyole eingesetzt, so erhält man Polyesteretherpolyole, die ebenfalls als Startersubstanzen zur Herstellung der Polyethercarbonatpolyole dienen können.

Des Weiteren können als H-funktionelle Startersubstanz Polycarbonatdiole eingesetzt werden, die beispielsweise durch Umsetzung von Phosgen, Dimethylcarbonat, Diethylcarbonat oder Diphenylcarbonat und difunktionellen Alkoholen oder Polyesterpolyolen oder Polyetherpolyolen hergestellt werden. Beispiele zu Polycarbonaten finden sich z. B. in der EP-A 1359177.

In einer weiteren Ausführungsform der Erfindung können Polyethercarbonatpolyole als H-funktionelle Startersubstanz eingesetzt werden. Insbesondere werden Polyethercarbonatpolyole, die nach dem hier beschriebenen erfindungsgemäßen Verfahren erhältlich sind, eingesetzt. Diese als H-funktionelle Startersubstanz eingesetzten Polyethercarbonatpolyole werden hierzu in einem separaten Reaktionsschritt zuvor hergestellt.

Die H-funktionelle Startersubstanz weist im Allgemeinen eine Funktionalität (d.h. Anzahl an für die Polymerisation aktiven H-Atomen pro Molekül) von 1 bis 8, bevorzugt von 2 oder 3 auf. Die H-funktionelle Startersubstanz wird entweder einzeln oder als Gemisch aus mindestens zwei H-funktionellen Startersubstanzen eingesetzt.

Besonders bevorzugt handelt es sich bei der H-funktionellen Startersubstanz um mindestens eine Verbindungen ausgewählt aus der Gruppe bestehend aus Ethylenglykol, Propylenglykol, 1,3-Propandiol, 1,3-Butandiol, 1,4-Butandiol, 1,5-Pentandiol, 2-Methylpropan-1,3-diol, Neopentylglykol, 1,6-Hexandiol, 1,8-Octandiol, Diethylenglykol, Dipropylenglykol, Glycerin, Trimethylolpropan, Pentaerythrit, Sorbitol, Polyethercarbonatpolyole mit einem Molekulargewicht $M_n$ im Bereich von 150 bis 8000 g/mol bei einer Funktionalität von 2 bis 3 und Polyetherpolyole mit einem Molekulargewicht $M_n$ im Bereich von 150 bis 8000 g/mol und einer Funktionalität von 2 bis 3.

In einer besonders bevorzugten Ausführungsform ist in Schritt (α) die Teilmenge H-funktioneller Startersubstanz ausgewählt aus mindestens einer Verbindung der Gruppe bestehend aus Polyethercarbonatpolyolen mit einem Molekulargewicht $M_n$ im Bereich von 150 bis 8000 g/mol bei einer Funktionalität von 2 bis 3 und Polyetherpolyolen mit einem

Molekulargewicht $M_n$ im Bereich von 150 bis 8000 g/mol und einer Funktionalität von 2 bis 3. In einer weiteren besonders bevorzugten Ausführungsform ist die H-funktionelle Startersubstanz in Schritt (γ) ausgewählt aus mindestens einer Verbindung der Gruppe bestehend aus Ethylenglykol, Propylenglykol, 1,3-Propandiol, 1,3-Butandiol, 1,4-Butandiol, 1,5-Pentandiol, 2-Methylpropan-1,3-diol, Neopentylglykol, 1,6-Hexandiol, 1,8-Octandiol, Diethylenglykol, Dipropylenglykol, Glycerin, Trimethylolpropan, Pentaerythrit und Sorbitol.

Die Herstellung der Polyethercarbonatpolyole erfolgt durch katalytische Anlagerung von Kohlendioxid und Alkylenoxid an H-funktionelle Startersubstanz. Unter "H-funktionell" wird im Sinne der Erfindung die Anzahl an für die Alkoxylierung aktiven H-Atomen pro Molekül der Startersubstanz verstanden.

Die H-funktionelle Startersubstanz, die während der Reaktion kontinuierlich in den Reaktor zudosiert wird, kann Komponente K enthalten.

Suspensionsmittel

Das gegebenenfalls eingesetzte Suspensionsmittel enthält keine H-funktionelle Gruppen. Als Suspensionsmittel, das keine H-funktionelle Gruppen aufweist, sind alle polar-aprotischen, schwach polar-aprotischen und unpolar-aprotischen Lösungsmittel geeignet, die jeweils keine H-funktionelle Gruppen enthalten. Als Suspensionsmittel, das keine H-funktionelle Gruppen aufweist, können auch eine Mischung aus zwei oder mehrerer dieser Suspensionsmittel eingesetzt werden. Beispielhaft seien an dieser Stelle folgende polar-aprotischen Lösungsmittel genannt: 4-Methyl-2-oxo-1,3-dioxolan (im Folgenden auch als cyclisches Propylencarbonat oder cPC bezeichnet), 1,3-Dioxolan-2-on (im Folgenden auch als cyclisches Ethylencarbonat oder cEC bezeichnet), Aceton, Methylethylketon, Acetonitril, Nitromethan, Dimethylsulfoxid, Sulfolan, Dimethylformamid, Dimethylacetamid und N-Methylpyrrolidon. Zu der Gruppe der unpolar- und schwach polar-aprotischen Lösungsmittel zählen z.B. Ether, wie z.B. Dioxan, Diethylether, Methyltert-Butylether und Tetrahydrofuran, Ester, wie z.B. Essigsäureethylester und Essigsäurebutylester, Kohlenwasserstoffe, wie z.B. Pentan, n-Hexan, Benzol und alkylierte Benzolderivate (z.B. Toluol, Xylol, Ethylbenzol) und chlorierte Kohlenwasserstoffe, wie z.B. Chloroform, Chlorbenzol, Dichlorbenzol und Tetrachlorkohlenstoff. Bevorzugt als Suspensionsmittel, das keine H-funktionelle Gruppen aufweist, werden 4-Methyl-2-oxo-1,3-dioxolan, 1,3-Dioxolan-2-on, Toluol, Xylol, Ethylbenzol, Chlorbenzol und Dichlorbenzol sowie Mischungen zweier oder mehrerer dieser Suspensionsmittel eingesetzt, besonders bevorzugt ist 4-Methyl-2-oxo-1,3-dioxolan und 1,3-Dioxolan-2-on oder eine Mischung aus 4-Methyl-2-oxo-1,3-dioxolan und 1,3-Dioxolan-2-on.

Bevorzugt wird in Schritt (γ) 2 Gew.-% bis 20 Gew.-%, besonders bevorzugt 5 Gew.-% bis 15 Gew.-% und insbesondere bevorzugt 7 Gew.-% bis 11 Gew.-% des Suspensionsmittels, das keine H-funktionelle Gruppen aufweist, zudosiert, bezogen auf die Summe der in Schritt (γ) zudosierten Komponenten.

Komponente K

Als Komponente K geeignete Verbindungen sind dadurch gekennzeichnet, dass sie mindestens eine Phosphor-Sauerstoff-Bindung enthalten. Als Komponente K geeignet sind beispielsweise Phosphorsäure sowie Phosphorsäuresalze, Phosporsäurehalogenide, Phosphorsäureamide, Phosphorsäureester und Salze der Mono- und Diester der Phosphorsäure.

Unter den unter Komponente K zuvor und nachfolgend genannten Estern werden im Sinne der Erfindung jeweils die Alkylester, Arylester und/oder Alkarylester-Derivate verstanden. Als Phosphorsäureester sind beispielsweise geeignet Mono-, Di- oder Triester von Phosphorsäure, Mono-, Di-, Tri- oder Tetraester von Pyrophosphorsäure und Mono-, Di-, Tri-, Tetra- oder Polyester von Polyphosphorsäure und Alkoholen mit 1 bis 30 C-Atomen. Als Komponente K sind beispielsweise die folgenden Verbindungen geeignet: Phosphorsäuretriethylester, Phosphorsäurediethylester, Phosphorsäuremonoethylester, Phosphorsäuretripropylester, Phosphorsäuredipropylester, Phosphorsäuremonopropylester, Phosphorsäuretributylester, Phosphorsäuredibutylester, Phosphorsäuremonobutylester, Phosphorsäuretrioctylester, Phosphorsäuretris(2-ethylhexyl)ester, Phosphorsäuretris-(2-butoxyethyl)ester, Phosphorsäurediphenylester, Phosphorsäuredikresylester, Fructose-1,6-bisphosphat, Glucose-1-phosphat, Phosphorsäure-bis-(dimethylamid)-chlorid, Phosphorsäure-bis-(4-nitrophenyl)-ester, Phosphorsäure-cyclopropylmethyl-diethyl-ester, Phosphorsäure-dibenzylester, Phosphorsäure-diethyl-3 -butenylester, Phosphorsäuredihexadecylester, Phosphorsäure-diisopropylester-chlorid, Phosphorsäure-diphenylester, Phosphorsäure-diphenylester-chlorid, Phosphorsäure-2-hydroxyethylmethacrylatester, Phosphorsäure-mono-(4-chlorphenylester)-dichlorid, Phosphorsäure-mono-(4-nitrophenylester)-dichlorid, Phosphorsäure-monophenylester-dichlorid, Phosphorsäuretridecylester, Phosphorsäure-trikresylester, Phosphorsäure-trimethylester, Phosphorsäuretriphenylester, Phosphorsäuretripyrolidid, Phosphorsulfochlorid, Phosphorsäure-dichloriddimethylamid, Phosphorsäuredichloridmethylester, Phosphorylbromid, Phosphorylchlorid, Phosphorylchinolinchlorid Calciumsalz und O-Phosphorylethanolamin, Alkali- und Ammoniumdihydrogenphosphate, Alkali-, Erdalkali- und Ammoniumhydrogenphosphate, Alkali-, Erdalkali- und Ammoniumphosphate.

Unter Ester der Phosphorsäure (Phosphorsäureester) werden auch die durch Propoxylierung der Phosphorsäure erhältlichen Produkte verstanden (bspw. erhältlich als Exolit® OP 560).

Als Komponente K geeignet sind auch Phosphonsäure und Phosphorige Säure sowie Mono- und Diester der Phosphonsäure sowie Mono-, Di- und Triester der Phosphorigen Säure sowie jeweils deren Salze, Halogenide und Amide.

Als Phosphonsäureester sind beispielsweise geeignet Mono- oder Diester von Phosphonsäure, Alkylphosphonsäuren, Arylphosphonsäuren, Alkoxycarbonylalkylphosphonsäuren, Alkoxycarbonylphosphonsäuren, Cyanalkylphosphonsäuren und Cyanphosphonsäuren oder Mono-, Di-, Tri- oder Tetraester von Alkyldiphosphonsäuren und Alkoholen mit 1 bis 30 C-Atomen. Als Phosphorigsäureester sind beispielsweise geeignet Mono-, Di- oder Triester der Phosphorigen Säure und Alkoholen mit 1 bis 30 C-Atomen. Das beinhaltet beispielsweise Phenylphosphonsäure, Butylphosphonsäure, Dodecylphosphonsäure, Ethylhexylphosphonsäure, Octylphosphonsäure, Ethylphosphonsäure, Methylphosphonsäure, Octadecylphosphonsäure sowie deren Mono- und Di-methylester, - ethylester, -butylester, -ethylhexylester oder -phenylester, Butylphosphonsäuredibutylester, Phenylphosphonsäuredioctylester, Phosphonoameisensäuretriethylester, Phosphonoessigsäuretrimethylester, Phosphonoessigsäuretriethylester, 2-Phosphonopropionsäuretrimethylester, 2-Phosphonopropionsäuretriethylester, 2-Phosphonopropionsäuretripropylester, 2-Phosphonopropionsäuretributylester, 3 -Phosphonopropionsäuretriethylester, 2-Phosphonobuttersäuretriethylester, 4-Phosphonocrotonsäuretriethylester, (12-Phosphonododecyl)-phosphonsäure, Phosphonoessigsäure, Phosphonoessigsäure-P,P-bis-(2,2,2-trifluorethyl)-methylester, Phosphonoessigsäure-P,P-diethyl-trimethylsilylester, Phosphonoessigsäure-P,P-dimethyl-tert.-butylester, Phosphonoessigsäure-P,P-dimethylester Kaliumsalz, Phosphonoessigsäure-P,P-dimethylethylester, 16-Phosphonohexadecansäure, 6-Phosphonohexansäure, N-(Phosphonomethyl)-glycin, N-(Phosphonomethyl)-glycin-monoisopropylaminsalz, N-(Phosphonomethyl)-iminodiessigsäure, (8-Phosphono-octyl)-phosphonsäure, 3-Phosphono-propionsäure, 11-Phosphonoundecansäure, Phosphonsäurepinakolester, Trilaurylphosphit, Tris-(3-ethyloxethanyl-3-methyl)phosphit, Hepta-kis(dipropylenglykol)phosphit, Phosphorigsäure-bis-(diisopropylamid)-2-cyanethylester, Phosphorigsäure-bis-(diisopropylamid)-methylester, Phosphorigsäuredibutylester, Phosphorigsäure-(diethylamid)-dibenzylester, Phosphorigsäure-(diethylamid)-ditertiärbutylester, Phosphorigsäure-diethylester, Phosphorigsäure-(diisopropylamind)-diallylester, Phosphorigsäure-(diisopropylamid)-dibenzylester, Phosphorigsäure-(diisopropylamid)-di-tert.-butylester, Phosphorigsäure-(diisopropylamid)-dimethylester, Phosphorigsäure-(dimethylamid)-dibenzylester, Phosphorigsäuredimethylester, Phosphorigsäure-dimethyl-trimethylsilylester, Phosphorigsäure-diphenylester, Phosphorigsäure-methylesterdichlorid, Phosphorigsäure-mono-(2-cyanethylester)-diisopropylamid-chlorid, Phosphorigsäure-(o-phenylenester)-chlorid, Phosphorigsäuretributylester, Phosphorigsäure-triethylester, Phosphorigsäure-triisopropylester, Phosphorigsäure-triphenylester, Phosphorigsäure-tris-(tert.-butyl-dimethylsilyl)-ester, Phosphorigsäure-(tris-1,1,1,3,3,3-hexafluor-2-propyl)-ester, Phosphorigsäure-tris-(trimethylsilyl)-ester, Phosphorigsäure-dibenzylester. Unter Ester der Phosphorigen Säure werden auch die durch Propoxylierung der Phosphorigen Säure erhältlichen Produkte verstanden (bspw. erhältlich als Exolit® OP 550).

Als Komponente K geeignet sind auch Phosphinsäure, Phosphonigsäure und Phosphinigsäure sowie jeweils deren Ester. Als Phosphinsäureester sind beispielsweise geeignet Ester von Phosphinsäure, Alkylphosphinsäuren, Dialkylphosphinsäuren oder Arylphosphinsäuren und Alkoholen mit 1 bis 30 C-Atomen. Als Phosphonigsäureester sind beispielsweise geeignet Mono- und Diester von Phosphonigsäure oder Arylphosphonigsäure und Alkoholen mit 1 bis 30 C-Atomen. Das beinhaltet beispielsweise Diphenylphosphinsäure oder 9,10-Dihydro-9-Oxa-10-Phosphaphenanthren-10-Oxid.

Die als Komponente K geeigneten Ester der Phosphorsäure, Phosphonsäure, Phosphorigen Säure, Phosphinsäure, Phosphonigsäure oder Phosphinigsäure werden in der Regel durch Umsetzung von Phosphorsäure, Pyrophosphorsäure, Polyphosphorsäuren, Phosphonsäure, Alkylphosphonsäuren, Arylphosphonsäuren, Alkoxycarbonylalkylphosphonsäuren, Alkoxycarbonylphosphonsäuren, Cyanalkylphosphonsäuren, Cyanphosphonsäure, Alkyldiphosphonsäuren, Phosphonigsäure, Phosphorige Säure, Phosphinsäure, Phosphinigsäure oder deren Halogenderivaten oder Phosphoroxiden mit Hydroxyverbindungen mit 1 bis 30 C-Atomen wie Methanol, Ethanol, Propanol, Butanol, Pentanol, Hexanol, Heptanol, Octanol, Nonanol, Dekanol, Dodekanol, Tridekanol, Tetradekanol, Pentadekanol, Hexadekanol, Heptadekanol, Octadekanol, Nonadekanol, Methoxymethanol, Ethoxymethanol, Propoxymethanol, Butoxymethanol, 2-Ethoxyethanol, 2-Propoxyethanol, 2-Butoxyethanol, Phenol, Hydroxyessigsäureethylester, Hydroxyessigsäurepropylester, Hydroxypropionsäureethylester, Hydroxypropionsäurepropylester, 1,2-Ethandiol, 1,2-Propandiol, 1,2,3-Trihydroxypropan, 1,1,1-Trimethylolpropan oder Pentaerythrit erhalten.

Als Komponente K geeignete Phosphinoxide enthalten eine oder mehrere Alkyl-, Aryl oder Aralkylgruppen mit 1 - 30 C-Atomen, die an den Phosphor gebunden sind. Bevorzugte Phosphinoxide haben die allgemeine Formel $R_3P=O$ wobei R eine Alkyl-, Aryl oder Aralkylgruppe mit 1 - 20 C-Atomen ist. Geeignete Phosphinoxide sind beispielsweise Trimethylphosphinoxid, Tri(n-butyl)phosphinoxid, Tri(n-octyl)phosphinoxid, Triphenylphosphinoxid, Methyldibenzylphosphinoxid und deren Mischungen.

Des Weiteren sind als Komponente K geeignet Verbindungen des Phosphors, die durch Reaktion mit OH-funktionellen Verbindungen (wie bspw. Wasser oder Alkoholen) eine oder mehrere P-O-Bindung(en) ausbilden können. Beispielsweise kommen als solche Verbindungen des Phosphors in Frage Phosphor-(V)-sulfid, Phosphortribromid,

Phosphortrichlorid und Phosphortriiodid. Es können auch beliebige Mischungen der vorgenannten Verbindungen als Komponente K eingesetzt werden. Besonders bevorzugt ist Komponente K Phosphorsäure.

DMC-Katalysatoren

DMC-Katalysatoren zur Verwendung in der Homopolymerisation von Alkylenoxiden sind im Prinzip aus dem Stand der Technik bekannt (siehe z.B. US-A 3 404 109, US-A 3 829 505, US-A 3 941 849 und US-A 5 158 922). DMC-Katalysatoren, die z.B. in US-A 5 470 813, EP-A 700 949, EP-A 743 093, EP-A 761 708, WO 97/40086, WO 98/16310 und WO 00/47649 beschrieben sind, besitzen eine sehr hohe Aktivität und ermöglichen die Herstellung von Polyethercarbonatpolyolen bei sehr geringen Katalysatorkonzentrationen, so dass eine Abtrennung des Katalysators aus dem fertigen Produkt i.a. nicht mehr erforderlich ist. Ein typisches Beispiel sind die in EP-A 700 949 beschriebenen hochaktiven DMC-Katalysatoren, die neben einer Doppelmetallcyanid-Verbindung (z.B. Zinkhexacyanocobaltat(III)) und einem organischen Komplexliganden (z.B. tert.-Butanol) noch einen Polyether mit einem zahlenmittlerem Molekulargewicht größer als 500 g/mol enthalten.

Die DMC-Katalysatoren werden vorzugsweise erhalten, indem man

(i) im ersten Schritt eine wässrige Lösung eines Metallsalzes mit der wässrigen Lösung eines Metallcyanidsalzes in Gegenwart eines oder mehrerer organischen Komplexliganden, z.B. eines Ethers oder Alkohols, umsetzt,
(ii) wobei im zweiten Schritt der Feststoff aus der aus (i) erhaltenen Suspension durch bekannte Techniken (wie Zentrifugation oder Filtration) abgetrennt wird,
(iii) wobei gegebenenfalls in einem dritten Schritt der isolierte Feststoff mit einer wässrigen Lösung eines organischen Komplexliganden gewaschen wird (z.B. durch Resuspendieren und anschließende erneute Isolierung durch Filtration oder Zentrifugation),
(iv) wobei anschließend der erhaltene Feststoff, gegebenenfalls nach Pulverisierung, bei Temperaturen von im allgemeinen 20 - 120 °C und bei Drücken von im Allgemeinen 0,1 mbar bis Normaldruck (1013 mbar) getrocknet wird,

und wobei im ersten Schritt oder unmittelbar nach der Ausfällung der Doppelmetallcyanidverbindung (zweiter Schritt) ein oder mehrere organische Komplexliganden, vorzugsweise im Überschuss (bezogen auf die Doppelmetallcyanidverbindung) und gegebenenfalls weitere komplexbildende Komponenten zugesetzt werden.

Die in den DMC-Katalysatoren enthaltenen Doppelmetallcyanid-Verbindungen sind die Reaktionsprodukte wasserlöslicher Metallsalze und wasserlöslicher Metallcyanidsalze. Beispielsweise werden eine wässrige Lösung von Zinkchlorid (bevorzugt im Überschuss bezogen auf das Metallcyanidsalz wie beispielsweise Kaliumhexacyanocobaltat) und Kaliumhexacyanocobaltat gemischt und anschließend Dimethoxyethan (Glyme) oder *tert*-Butanol (vorzugsweise im Überschuss, bezogen auf Zinkhexacyanocobaltat) zur gebildeten Suspension gegeben.

Zur Herstellung der Doppelmetallcyanid-Verbindungen geeignete Metallsalze besitzen bevorzugt die allgemeine Formel (II),

$$M(X)_n \qquad (II)$$

wobei

M ausgewählt ist aus den Metallkationen $Zn2^+$, $Fe^{2+}$, $Ni^{2+}$, $Mn2^+$, $Co^{2+}$, $Sr^{2+}$, $Sn^{2+}$, $Pb^{2+}$ und, $Cu2^+$, bevorzugt ist M $Zn^{2+}$, $Fe^{2+}$, $Co^{2+}$ oder $Ni^{2+}$,
X sind ein oder mehrere (d.h. verschiedene) Anionen, vorzugsweise ein Anion ausgewählt aus der Gruppe der Halogenide (d.h. Fluorid, Chlorid, Bromid, Iodid), Hydroxid, Sulfat, Carbonat, Cyanat, Thiocyanat, Isocyanat, Isothiocyanat, Carboxylat, Oxalat und Nitrat;
n ist 1, wenn X = Sulfat, Carbonat oder Oxalat ist und
n ist 2, wenn X = Halogenid, Hydroxid, Carboxylat, Cyanat, Thiocyanat, Isocyanat, Isothiocyanat oder Nitrat ist,

oder geeignete Metallsalze besitzen die allgemeine Formel (III),

$$M_r(X)_3 \qquad (III)$$

wobei

M ausgewählt ist aus den Metallkationen $Fe^{3+}$, $Al^{3+}$, $Co^{3+}$ und $Cr^{3+}$,
X sind ein oder mehrere (d.h. verschiedene) Anionen, vorzugsweise ein Anion ausgewählt aus der Gruppe der Halogenide (d.h. Fluorid, Chlorid, Bromid, Iodid), Hydroxid, Sulfat, Carbonat, Cyanat, Thiocyanat, Isocyanat, Isothiocyanat, Carboxylat, Oxalat und Nitrat;
r ist 2, wenn X = Sulfat, Carbonat oder Oxalat ist und
r ist 1, wenn X = Halogenid, Hydroxid, Carboxylat, Cyanat, Thiocyanat, Isocyanat, Isothiocyanat oder Nitrat ist,

oder geeignete Metallsalze besitzen die allgemeine Formel (IV),

$$M(X)_s \qquad (IV)$$

wobei

M ausgewählt ist aus den Metallkationen $Mo^{4+}$, $V^{4+}$ und $W^{4+}$
X sind ein oder mehrere (d.h. verschiedene) Anionen, vorzugsweise ein Anion ausgewählt aus der Gruppe der Halogenide (d.h. Fluorid, Chlorid, Bromid, Iodid), Hydroxid, Sulfat, Carbonat, Cyanat, Thiocyanat, Isocyanat, Isothiocyanat, Carboxylat, Oxalat und Nitrat;
s ist 2, wenn X = Sulfat, Carbonat oder Oxalat ist und
s ist 4, wenn X = Halogenid, Hydroxid, Carboxylat, Cyanat, Thiocyanat, Isocyanat, Isothiocyanat oder Nitrat ist,

oder geeignete Metallsalze besitzen die allgemeine Formel (V),

$$M(X)_t \qquad (V)$$

wobei

M ausgewählt ist aus den Metallkationen $Mo^{6+}$ und $W^{6+}$
X sind ein oder mehrere (d.h. verschiedene) Anionen, vorzugsweise ein Anion ausgewählt aus der Gruppe der Halogenide (d.h. Fluorid, Chlorid, Bromid, Iodid), Hydroxid, Sulfat, Carbonat, Cyanat, Thiocyanat, Isocyanat, Isothiocyanat, Carboxylat, Oxalat und Nitrat;
t ist 3, wenn X = Sulfat, Carbonat oder Oxalat ist und
t ist 6, wenn X = Halogenid, Hydroxid, Carboxylat, Cyanat, Thiocyanat, Isocyanat, Isothiocyanat oder Nitrat ist,

Beispiele geeigneter Metallsalze sind Zinkchlorid, Zinkbromid, Zinkjodid, Zinkacetat, Zinkacetylacetonat, Zinkbenzoat, Zinknitrat, Eisen(II)sulfat, Eisen(II)bromid, Eisen(II)-chlorid, Eisen(III)chlorid, Cobalt(II)chlorid, Cobalt(II)thiocyanat, Nickel(II)chlorid und Nickel(II)nitrat. Es können auch Mischungen verschiedener Metallsalze eingesetzt werden. Zur Herstellung der Doppelmetallcyanid-Verbindungen geeignete Metallcyanidsalze besitzen bevorzugt die allgemeine Formel (VI)

$$(Y)_a\, M'(CN)_b\, (A)_c \qquad (VI)$$

wobei

M' ausgewählt ist aus einem oder mehreren Metallkationen der Gruppe bestehend aus Fe(II), Fe(III), Co(II), Co(III), Cr(II), Cr(III), Mn(II), Mn(III), Ir(III), Ni(II), Rh(III), Ru(II), V(IV) und V(V), bevorzugt ist M' ein oder mehrere Metallkationen der Gruppe bestehend aus Co(II), Co(III), Fe(II), Fe(III), Cr(III), Ir(III) und Ni(II),
Y ausgewählt ist aus einem oder mehreren Metallkationen der Gruppe bestehend aus Alkalimetall (d.h. $Li^+$, $Na^+$, $K^+$, $Rb^+$)und Erdalkalimetall (d.h. $Be^{2+}$, $Mg^{2+}$, $Ca^{2+}$, $Sr^{2+}$, $Ba^{2+}$), A ausgewählt ist aus einem oder mehreren Anionen der Gruppe bestehend aus Halogenide (d.h. Fluorid, Chlorid, Bromid, Iodid), Hydroxid, Sulfat, Carbonat, Cyanat, Thiocyanat, Isocyanat, Isothiocyanat, Carboxylat, Azid, Oxalat oder Nitrat und
a, b und c sind ganzzahlige Zahlen, wobei die Werte für a, b und c so gewählt sind, dass die Elektroneutralität des Metallcyanidsalzes gegeben ist; a ist vorzugsweise 1, 2, 3 oder 4; b ist vorzugsweise 4, 5 oder 6; c besitzt bevorzugt den Wert 0.

Beispiele geeigneter Metallcyanidsalze sind Natriumhexacyanocobaltat(III), Kaliumhexacyanocobaltat(III), Kaliumhexacyanoferrat(II), Kaliumhexacyanoferrat(III), Calcium-hexacyanocobaltat(III) und Lithiumhexacyanocobaltat(III).
Bevorzugte Doppelmetallcyanid-Verbindungen, die in den DMC-Katalysatoren enthalten sind, sind Verbindungen der allgemeinen Formel (VII)

$$M_x[M'_{x'}(CN)_y]_z \qquad (VII)$$

worin M wie in Formel (II) bis (V) und
M' wie in Formel (VI) definiert ist, und
x, x', y und z sind ganzzahlig und so gewählt, dass die Elektronenneutralität der Doppelmetallcyanidverbindung gegeben ist.

Vorzugsweise ist
x = 3, x' = 1, y = 6 und z = 2,
M = Zn(II), Fe(II), Co(II) oder Ni(II) und
M' = Co(III), Fe(III), Cr(III) oder Ir(III).

Beispiele geeigneter Doppelmetallcyanidverbindungen a) sind Zinkhexacyanocobaltat(III), Zinkhexacyanoiridat(III), Zinkhexacyanoferrat(III) und Cobalt(II)hexacyanocobaltat(III).

Weitere Beispiele geeigneter Doppelmetallcyanid-Verbindungen sind z.B. US 5 158 922 (Spalte 8, Zeilen 29 - 66) zu entnehmen. Besonders bevorzugt verwendet wird Zinkhexacyanocobaltat(III).

Die bei der Herstellung der DMC-Katalysatoren zugesetzten organischen Komplexliganden sind beispielsweise in US 5 158 922 (siehe insbesondere Spalte 6, Zeilen 9 bis 65), US 3 404 109, US 3 829 505, US 3 941 849, EP-A 700 949, EP-A 761 708, JP 4 145 123, US 5 470 813, EP-A 743 093 und WO-A 97/40086) offenbart. Beispielsweise werden als organische Komplexliganden wasserlösliche, organische Verbindungen mit Heteroatomen, wie Sauerstoff, Stickstoff, Phosphor oder Schwefel, die mit der Doppelmetallcyanid-Verbindung Komplexe bilden können, eingesetzt. Bevorzugte organische Komplexliganden sind Alkohole, Aldehyde, Ketone, Ether, Ester, Amide, Harnstoffe, Nitrile, Sulfide und deren Mischungen. Besonders bevorzugte organische Komplexliganden sind aliphatische Ether (wie Dimethoxyethan), wasserlösliche aliphatische Alkohole (wie Ethanol, Isopropanol, n-Butanol, iso-Butanol, sek.-Butanol, tert-Butanol, 2-Methyl-3-buten-2-ol und 2-Methyl-3-butin-2-ol), Verbindungen, die sowohl aliphatische oder cycloaliphatische Ethergruppen wie auch aliphatische Hydroxylgruppen enthalten (wie z.B. Ethylenglykol-mono-tert.-butylether, Diethylenglykol-mono-tert.-butylether, Tripropylenglykol-mono-methylether und 3-Methyl-3-oxetan-methanol). Höchst bevorzugte organische Komplexliganden sind ausgewählt aus einer oder mehrerer Verbindungen der Gruppe bestehend aus Dimethoxyethan, tert-Butanol 2-Methyl-3-buten-2-ol, 2-Methyl-3-butin-2-ol, Ethylenglykol-mono-tert.-butylether und 3-Methyl-3-oxetan-methanol.

Optional werden bei der Herstellung der DMC-Katalysatoren eine oder mehrere komplexbildende Komponente(n) aus den Verbindungsklassen der Polyether, Polyester, Polycarbonate, Polyalkylenglyko lsorbitanester, Polyalkylenglykolglycidylether, Polyacrylamid, Poly(acrylamid-co-acrylsäure), Polyacrylsäure, Poly(acrylsäure-co-maleinsäure), Polyacrylnitril, Polyalkylacrylate, Polyalkylmethacrylate, Polyvinylmethylether, Polyvinylethylether, Polyvinylacetat, Polyvinylalkohol, Poly-N-vinylpyrrolidon, Poly(N-vinylpyrrolidon-co-acrylsäure), Polyvinylmethylketon, Poly(4-vinylphenol), Poly-(acrylsäure-co-styrol), Oxazolinpolymere, Polyalkylenimine, Maleinsäure- und Maleinsäureanhydridcopolymere, Hydroxyethylcellulose und Polyacetale, oder der Glycidylether, Glycoside, Carbonsäureester mehrwertiger Alkohole, Gallensäuren oder deren Salze, Ester oder Amide, Cyclodextrine, Phosphorverbindungen, $\alpha,\beta$-ungesättigten Carbonsäureester oder ionische oberflächen- bzw. grenzflächenaktiven Verbindungen eingesetzt.

Bevorzugt werden bei der Herstellung der DMC-Katalysatoren im ersten Schritt die wässrigen Lösungen des Metallsalzes (z.B. Zinkchlorid), eingesetzt im stöchiometrischen Überschuss (mindestens 50 Mol-%) bezogen auf Metallcyanidsalz (also mindestens ein molares Verhältnis von Metallsalz zu Metallcyanidsalz von 2,25 zu 1,00) und des Metallcyanidsalzes (z.B. Kaliumhexacyanocobaltat) in Gegenwart des organischen Komplexliganden (z.B. tert.-Butanol) umgesetzt, wobei sich eine Suspension bildet, die die Doppelmetallcyanid-Verbindung (z.B. Zinkhexacyanocobaltat), Wasser, überschüssiges Metallsalz, und den organischen Komplexliganden enthält.

Der organische Komplexligand kann dabei in der wässrigen Lösung des Metallsalzes und/oder des Metallcyanidsalzes vorhanden sein, oder er wird der nach Ausfällung der Doppelmetallcyanid-Verbindung erhaltenen Suspension unmittelbar zugegeben. Es hat sich als vorteilhaft erwiesen, die wässrigen Lösungen des Metallsalzes und des Metallcyanidsalzes, und den organischen Komplexliganden unter starkem Rühren zu vermischen. Optional wird die im ersten Schritt gebildete Suspension anschließend mit einer weiteren komplexbildenden Komponente behandelt. Die komplexbildende Komponente wird dabei bevorzugt in einer Mischung mit Wasser und organischem Komplexliganden eingesetzt. Ein bevorzugtes Verfahren zur Durchführung des ersten Schrittes (d.h. der Herstellung der Suspension) erfolgt unter Einsatz einer Mischdüse, besonders bevorzugt unter Einsatz eines Strahldispergators wie in WO-A 01/39883 beschrieben.

Im zweiten Schritt erfolgt die Isolierung des Feststoffs (d.h. die Vorstufe des Katalysators) aus der Suspension durch bekannte Techniken, wie Zentrifugation oder Filtration.

In einer bevorzugten Ausführungsvariante wird der isolierte Feststoff anschließend in einem dritten Verfahrensschritt mit einer wässrigen Lösung des organischen Komplexliganden gewaschen (z.B. durch Resuspendieren und anschließende erneute Isolierung durch Filtration oder Zentrifugation). Auf diese Weise können zum Beispiel wasserlösliche Nebenprodukte, wie Kaliumchlorid, aus dem Katalysator entfernt werden. Bevorzugt liegt die Menge des organischen Komplexliganden in der wässrigen Waschlösung zwischen 40 und 80 Gew.-%, bezogen auf die Gesamtlösung.

Optional wird im dritten Schritt der wässrigen Waschlösung weitere komplexbildende Komponente, bevorzugt im Bereich zwischen 0,5 und 5 Gew.-%, bezogen auf die Gesamtlösung, zugefügt.

Außerdem ist es vorteilhaft, den isolierten Feststoff mehr als einmal zu waschen. Vorzugsweise wird in einem ersten Waschschritt (iii-1) mit einer wässrigen Lösung des organischen Komplexliganden gewaschen (z.B. durch Resuspendieren und anschließende erneute Isolierung durch Filtration oder Zentrifugation), um auf diese Weise zum Beispiel wasserlösliche Nebenprodukte, wie Kaliumchlorid, aus dem Katalysator zu entfernen. Besonders bevorzugt liegt die Menge des organischen Komplexliganden in der wässrigen Waschlösung zwischen 40 und 80 Gew.-%, bezogen auf die Gesamtlösung des ersten Waschschritts. In den weiteren Waschschritten (iii-2) wird entweder der erste Waschschritt einmal oder mehrmals, vorzugsweise einmal bis dreimal wiederholt, oder vorzugsweise wird eine nicht-wässrige Lösung, wie z.B. eine Mischung oder Lösung aus organischem Komplexliganden und weiterer komplexbildender Komponente (bevorzugt im Bereich zwischen 0,5 und 5 Gew.-%, bezogen auf die Gesamtmenge der Waschlösung des Schrittes (iii-2)), als Waschlösung eingesetzt und der Feststoff damit einmal oder mehrmals, vorzugsweise einmal bis dreimal gewaschen.

Der isolierte und gegebenenfalls gewaschene Feststoff wird anschließend, gegebenenfalls nach Pulverisierung, bei Temperaturen von im allgemeinen 20 - 100 °C und bei Drücken von im Allgemeinen 0,1 mbar bis Normaldruck (1013 mbar) getrocknet.

Ein bevorzugtes Verfahren zur Isolierung der DMC-Katalysatoren aus der Suspension durch Filtration, Filterkuchenwäsche und Trocknung wird in WO-A 01/80994 beschrieben. Neben den bevorzugt eingesetzten DMC-Katalysatoren auf Basis von Zinkhexacyanocobaltat ($Zn_3[Co(CN)_6]_2$) können für das erfindungsgemäße Verfahren auch andere dem Fachmann aus dem Stand der Technik für die Copolymerisation von Epoxiden und Kohlendioxid bekannte Metallkomplexkatalysatoren auf Basis der Metalle Zink und/oder Cobalt eingesetzt werden. Dies beinhaltet insbesondere sogenannte Zink-Glutarat-Katalysatoren (beschrieben z.B. in M. H. Chisholm et al., Macromolecules 2002, 35, 6494), sogenannte Zink-Diiminat-Katalysatoren (beschrieben z.B. in S. D. Allen, J. Am. Chem. Soc. 2002, 124, 14284), sogenannte Cobalt-Salen-Katalysatoren (beschrieben z.B. in US 7,304,172 B2, US 2012/0165549 A1) und bimetallische Zink-Komplexe mit makrozyklischen Liganden (beschrieben z.B. in M. R. Kember et al., Angew. Chem., Int. Ed., 2009, 48, 931).

Aus Schritt (1-i) werden, wie in Schema (I) dargestellt, Polyethercarbonatpolyol und, entsprechend dem in Schritt (1-i) eingesetzten Alkylenoxid, cyclisches Carbonat erhalten. Bevorzugt ist das cyclische Carbonat ausgewählt aus mindestens einer Verbindung der Gruppe bestehend aus cyclisches Propylencarbonat und cyclisches Ethylencarbonat.

Schritt (1-ii):

Von dem aus Schritt (1-i) erhaltenen Reaktionsgemisch wird in Schritt (1-ii) cyclisches Carbonat abgetrennt. Die Abtrennung des cyclischen Carbonats kann nach dem Fachmann bekannten Verfahren erfolgen. Bevorzugt werden dabei thermische Verfahren wie z.B. Destillation oder Rektifikation eingesetzt. Besonders bevorzugt wird das cyclische Carbonat durch eine Verdampfungseinrichtung, wie z.B. Dünnschichtverdampfer oder Fallfilmverdampfer, eine Strippingkolonne oder einer Kombination der genannten vom Reaktionsgemisch aus Schritt (1-i) abgetrennt. Die Abtrennung des cyclischen Carbonats kann dabei nach einem einstufigen oder mehrstufigen Verfahren erfolgen, wobei ein zweistufiges Verfahren bevorzugt ist. Höchst bevorzugt erfolgt die Abtrennung des cyclischen Carbonats in einem zweistufigen Verfahren durch einen Dünnschichtverdampfer oder einen Fallfilmverdampfer in Kombination mit einer Strippingkolonne. Wird eine Verdampfungseinrichtung in Schritt (1-ii) eingesetzt, so kann diese bei einem Druck von 100 mbar oder darunter, bevorzugt bei 1 bis 100 mbar, besonders bevorzugt bei 1 bis 50 mbar, insbesondere bevorzugt bei 2 bis 25 mbar betrieben werden, wobei die Temperatur des aus Schritt (1-i) erhaltenen Reaktionsgemischs in Schritt (1-ii) bevorzugt 120 bis 180 °C, besonders bevorzugt 150 bis 170 °C beträgt. Bei dem Einsatz einer Strippingkolonne kann diese unter Normaldruck oder unter Vakuum, wie beispielsweise bei einem Druck von 1 bis 150 mbar, bevorzugt 50 bis 120 mbar betrieben werden, wobei die Strippingkolonne bei einer Temperatur von 120 bis 180 °C, bevorzugt 150 bis 170 °C betrieben werden kann.

Eine Apparatur zur Abtrennung von cyclischem Carbonat aus einem Gemisch enthaltend Polyethercarbonatpolyol ist beispielsweise in der Anmeldung EP 3 164 443 A1 offenbart. Das so erhaltene cyclische Propylencarbonat kann zur Entfernung von flüchtigen Nebenkomponenten, beispielsweise von Dimethyldioxanen, vor Schritt (1-iii) destillativ gereinigt werden.

Schritt (1-iii):

Das aus Schritt (1-ii) erhaltene cyclische Carbonat wird anschließend mit Wasser und einem Hydrolyse-Katalysator in einen Reaktor vorgelegt, erwärmt und hydrolytisch zu Kohlendioxid und Diol gespalten. Das entstehende $CO_2$ wird aus dem Reaktor abgelassen und kann gegebenenfalls wieder in Schritt (1-i) eingesetzt werden. Die Reaktion wird so lange fortgeführt, bis die CO2-Entwicklung zum Erliegen kommt. Es ist ebenso möglich während der Reaktion weiteres

cyclisches Carbonat aus Schritt (1-ii) in den Reaktor zu überführen.

Das molare Verhältnis von cyclischem Carbonat zu Wasser in Schritt (1-iii) beträgt bevorzugt 1:1 bis 1:10, besonders bevorzugt 1:1,2 bis 1:6 und insbesondere bevorzugt 1:1,5 bis 1:4. Die hydrolytische Spaltung des cyclischen Carbonats wird bevorzugt bei einer Temperatur von mindestens 40 °C, besonders bevorzugt bei mindestens 80 °C durchgeführt.

Als Hydrolyse-Katalysator können in Schritt (1-iii) dem Fachmann für die Hydrolyse bekannte katalytisch wirkende Verbindungen eingesetzt werden. Beispiele für solche Verbindungen sind Alkalimetallsalze, wie Alkalimetallhydroxide oder Alkalimetallcarbonate, Erdalkalimetallsalze, wie Erdalkalimetallhydroxide oder Erdalkalimetallcarbonate, oder Hydrolasen. In Schritt (1-iii) wird bevorzugt als Hydrolyse-Katalysator mindestens eine Verbindung ausgewählt aus der Gruppe bestehend aus Alkalimetallhydroxiden, Erdalkalimetallhydroxiden und Hydrolasen eingesetzt. Besonders bevorzugt ist die Verbindung ausgewählt aus der Gruppe der Alkalimetallhydroxide, insbesondere bevorzugt aus der Gruppe bestehend aus Kaliumhydroxid und Natriumhydroxid. Höchst bevorzugt wird als Hydrolyse-Katalysator in Schritt (1-iii) nur Kaliumhydroxid und/oder Natriumhydroxid eingesetzt.

Es werden üblicherweise 0,05 bis 1 Gew.-% bezogen auf das in Schritt (1-iii) eingesetzte cyclische Carbonat, an Hydrolyse-Katalysator eingesetzt. Es können aber auch weniger als 0,05 oder mehr als 1 Gew.-% eingesetzt werden.

In einer besonderen Ausführungsform wird in Schritt (1-iii) als Hydrolyse-Katalysator 0,05 bis 1 Gew.-%, bezogen auf das in Schritt (1-iii) eingesetzte cyclische Carbonat, mindestens eine Verbindung der Gruppe der Alkalimetallhydroxide eingesetzt.

In einer weiteren besonderen Ausführungsform wird in Schritt (1-iii) als Hydrolyse-Katalysator 0,05 bis 1 Gew.-%, bezogen auf das in Schritt (1-iii) eingesetzte cyclische Carbonat, mindestens eine Verbindung ausgewählt aus der Gruppe bestehend aus Kaliumhydroxid und Natriumhydroxid eingesetzt.

<u>Schritt (1-iv):</u>

Gegebenenfalls kann das in Schritt (1-iii) erhaltene Diol durch Destillation gereinigt werden. Die Destillation kann dabei unter Normaldruck oder reduziertem Druck erfolgen, wobei ein Druck von 150 mbar oder darunter eingestellt werden kann, bevorzugt 100 mbar oder darunter, besonders bevorzugt 50 mbar und darunter, insbesondere bevorzugt 20 mbar und darunter. Der Schritt (1-iv) kann in einem einstufigen oder mehrstufigen Verfahren durchgeführt werden, wobei im Sinne der Erfindung die Verwendung von Kolonnen, wie z.B. Füllkörperkolonnen, bei der Destillation als ein mehrstufiges Verfahren betrachtet wird.

Das vom Diol abgetrennte Gemisch enthaltend Hydrolyse-Katalysator kann gegebenenfalls wieder in Schritt (1-iii) eingesetzt werden.

<u>Schritt (2-i):</u>

In einer zweiten Verfahrensstufe wird vorzugsweise Polyol erhalten durch

a) Anlagerung von Alkylenoxid und gegebenenfalls Kohlendioxid, cyclisches Carbonsäureanhydrid und/oder cyclische Ester an das in der ersten Verfahrensstufe resultierende Diol und gegebenenfalls weiterer H-funktioneller Startersubstanz
oder
b) Umsetzung von Carbonsäure, cyclischem Carbonsäureanhydrid, nicht-cyclischem Ester und/oder cyclischem Ester mit dem in der ersten Verfahrensstufe resultierenden Diol und gegebenenfalls weiteren Alkoholen.

Als Polyol werden beispielsweise Polyetherpolyole, Polyesterpolyole, Polyethercarbonatpolyole oder Polyetheresterpolyole erhalten. Bevorzugt ist das Polyol mindestens eine Verbindung ausgewählt aus der Gruppe bestehend aus Polyetherpolyol, Polyesterpolyol und Polyethercarbonatpolyol.

Polyethercarbonatpolyole werden durch Anlagerung von Alkylenoxid und Kohlendioxid an das aus der ersten Verfahrensstufe erhaltene Diol und gegebenenfalls weiterer H-funktioneller Startersubstanz in Gegenwart eines Katalysators erhalten. Bevorzugt werden Polyethercarbonatpolyole durch das in Schritt (1-i) beschriebene Verfahren erhalten, wobei die eingesetzte H-funktionelle Startersubstanz das aus dem ersten Verfahrensschritt erhaltene Diol enthält.

Die erfindungsgemäß hergestellten Polyetherpolyole werden durch Anlagerung von Alkylenoxid an das in der ersten Verfahrensstufe resultierende Diol und gegebenenfalls weiterer H-funktioneller Startersubstanz nach dem Fachmann bekannten Herstellungsmethoden erhalten. Die als Alkylenoxid und gegebenenfalls weitere H-funktionelle Startersubstanz eingesetzten Verbindungen sind bereits in Schritt (1-i) beschrieben. Vorzugsweise werden neben dem aus dem ersten Verfahrensschritt erhältlichen Diol als zusätzliche H-funktionelle Startersubstanz zwei oder mehrwertige Alkohole, wie Ethandiol, 1,2- und 1,3-Propandiol, Diethylenglykol, Dipropylenglykol, 1,4-Butandiol, 1,6-Hexandiol, Triethanolamin, Bisphenole, Glycerin, Trimethylolpropan, Pentaerythrit, Sorbit oder Saccharose eingesetzt.

Als Katalysatoren für die Herstellung von Polyetherpolyolen im zweiten Verfahrensschritt können beispielsweise Alkalihydroxide, wie Natrium- oder Kaliumhydroxid, Alkalialkoholate, wie Natriummethylat, Natrium- oder Kaliumethylat oder Kaliumisopropylat, oder aminische Alkoxylierungs-Katalysatoren, wie Dimethylethanolamin (DMEOA), Imidazol und/oder Imidazolderivate, oder DMC-Katalysatoren, wie in Schritt (1-i) beschrieben, eingesetzt werden.

Die Polyesterpolyole können beispielsweise Polykondensate von dem aus der ersten Verfahrensstufe erhaltenen Diol und gegebenenfalls weiteren Alkoholen, mit 2 bis 12 Kohlenstoffatomen, vorzugsweise mit 2 bis 6 Kohlenstoffatomen, und Carbonsäuren, cyclischen Carbonsäureanhydriden, nicht-cyclischen Estern und/oder cyclischen Estern sein. Carbonsäuren sind beispielsweise Polycarbonsäuren, wie z.B. Di-, Tri- oder sogar Tetracarbonsäuren, oder Hydroxycarbonsäuren, bevorzugt werden aromatische und/oder aliphatische Dicarbonsäuren verwendet. Anstelle der freien Polycarbonsäuren können auch die entsprechenden Polycarbonsäureanhydride oder entsprechende Polycarbonsäureester von niederen Alkoholen zur Herstellung der Polyester verwendet werden. Die zur Herstellung von Polyesterpolyolen eingesetzten Ausgangsstoffe können auch biologischen Ursprungs sein und/oder durch fermentative Verfahren erhalten werden.

Als Carbonsäuren kommen insbesondere in Betracht: Bernsteinsäure, Glutarsäure, Adipinsäure, Korksäure, Azelainsäure, Sebazinsäure, Decandicarbonsäure, Tetrahydrophthalsäure, Hexahydrophthalsäure, Cyclohexandicarbonsäure, Tetrachlorphthalsäure, Itaconsäure, Malonsäure, 2-Methylbernsteinsäure, 3,3-Diethylglutarsäure, 2,2-Dimethylbernsteinsäure, Dodekandisäure, Endomethylentetrahydrophthalsäure, Dimerfettsäure, Trimerfettsäure, Zitronensäure, Trimellithsäure, Benzoesäure, Trimellitsäure, Maleinsäure, Fumarsäure, Phthalsäure, Isophthalsäure und Terephthalsäure. Verwendet werden können ebenso Derivate dieser Carbonsäuren, wie beispielsweise Dimethylterephthalat. Die Carbonsäuren können dabei sowohl einzeln als auch im Gemisch verwendet werden. Als Carbonsäuren werden bevorzugt Phthalsäure, Terephthalsäure, Glutarsäure Adipinsäure, Sebacinsäure und/oder Bernsteinsäure, besonders bevorzugt Adipinsäure, Phthalsäure und/oder Bernsteinsäure, verwendet.

Hydroxycarbonsäuren, die als Reaktionsteilnehmer bei der Herstellung eines Polyesterpolyols mit endständigen Hydroxylgruppen mitverwendet werden können, sind beispielsweise Hydroxycapronsäure, Hydroxybuttersäure, Hydroxydecansäure, Hydroxystearinsäure, Ricinolsäure und dergleichen. Geeignete Lactone sind unter anderem Caprolacton, Butyrolacton und Homologe.

Zur Herstellung der Polyesterpolyole kommen insbesondere auch biobasierte Ausgangsstoffe und/oder deren Derivate in Frage, wie z. B. Rizinusöl, Polyhydroxyfettsäuren, Ricinolsäure, Hydroxyl-modifizierte Öle, Weintraubenkernöl, schwarzem Kümmelöl, Kürbiskernöl, Borretschsamenöl, Sojabohnenöl, Weizensamenöl, Rapsöl, Sonnenblumenkernöl, Erdnussöl, Aprikosenkernöl, Pistazienöl, Mandelöl, Olivenöl, Macadamianussöl, Avocadoöl, Sanddornöl, Sesamöl, Hanföl, Haselnussöl, Primelöl, Wildrosenöl, Distelöl, Walnussöl, Fettsäuren, hydroxylmodifizierte und epoxidierte Fettsäuren und Fettsäureester, beispielsweise basierend auf Myristoleinsäure, Palmitoleinsäure, Ölsäure, Vaccensäure, Petroselinsäure, Gadoleinsäure, Erukasäure, Nervonsäure, Linolsäure, alpha- und gamma-Linolensäure, Stearidonsäure, Arachidonsäure, Timnodonsäure, Clupanodonsäure und Cervonsäure. Insbesondere bevorzugt sind Ester der Rizinolsäure mit mehrfunktionellen Alkoholen, z.B. Glycerin. Bevorzugt ist auch die Verwendung von Mischungen solcher biobasierten Säuren mit anderen Carbonsäuren, z.B. Phthalsäuren.

Als cyclische Carbonsäureanhydride können beispielsweise Phthalsäureanhydrid, Maleinsäureanhydrid, Bernsteinsäureanhydrid, Tetrahydrophthalsäureanhydrid, Hexahydrophthalsäureanhydrid, Glutarsäureanhydrid und/oder Dodecenylbernsteinsäureanhydrid eingesetzt werden, bevorzugt sind Phthalsäureanhydrid, Maleinsäureanhydrid, Bernsteinsäureanhydrid und Mischungen aus den genannten cyclischen Carbonsäureanhydriden.

Nicht-cyclische Ester können beispielsweise Terephthalsäuredimethylester, Bernsteinsäurediethylester, Bernsteinsäuredimethylester, Ethylmethylmalonat, Diethylmalonat, Adipinsäuredimethylester und/oder Adipinsäurediethylester sein, wobei Terephthalsäuredimethylester bevorzugt ist.

Cyclische Ester können beispielsweise Lactide, aliphatische oder aromatische Lactone sein. Die aliphatischen oder aromatischen Lactone können beispielsweise 4-gliedrige Lactone wie β-Propiolacton, β-Butyrolacton, β-Isovalerolacton, β-Caprolacton, β-Isocaprolacton und β-Methyl-β-valerolacton, oder 5-gliedrige Lactone, wie γ-Butyrolacton, γ-Valerolacton, 5-Methylfuran-2(3H)-on, 5-Methylidenedihydrofuran-2(3H)-on, 5-Hydroxyfuran-2(5H)-on, 2-Benzofuran-1(3H)-on und 6-Methy-2-benzofuran-1(3H)-on, oder 6-gliedrige Lactone, wie δ-Valerolacton, 1,4-Dioxan-2-on, Dihydrocumarin, 1H-Isochromen-1-on, 8H-pyrano[3,4-b]pyridine-8-on, 1,4-Dihydro-3H-isochromen-3-on, 7,8-Dihydro-5H-pyrano[4,3-b]pyridine-5-on, 4-Methyl-3,4-dihydro-1H-pyrano[3,4-b]pyridine-1-on, 6-Hydroxy-3,4-dihydro-1H-isochromen-1-on, 7-Hydroxy-3,4-dihydro-2H-chromen-2-on, 3-Ethyl-1H-isochromen-1-on, 3-(Hydroxymethyl)-1H-isochromen-1-on, 9-Hydroxy-1H,3H-benzo[de]isochromen-1-on, 6,7-Dimethoxy-1,4-dihydro-3H-isochromen-3-on und 3-Phenyl-3,4-dihydro-1H-isochromen-1-on, oder 7-gliedrige Lactone, wie ε-Caprolacton, 1,5-Dioxepan-2-on, 5-Methyloxepan-2-on, Oxepane-2,7-dion, thiepan-2-on, 5-Chlorooxepan-2-on, (4S)-4-(Propan-2-yl)oxepan-2-on, 7-Butyloxepan-2-on, 5-(4-Aminobuthyl)oxepan-2-on, 5-Phenyloxepan-2-on, 7-Hexyloxepan-2-on, (5S,7S)-5-Methyl-7-(propan-2-yl)oxepan-2-on und 4-Methyl-7-(propan-2-yl)oxepan-2-on, oder höhergliedrige Lactone, wie (7*E*)-Oxacycloheptadec-7-en-2-on sein. Besonders bevorzugt ist ε-Caprolacton und Dihydrocumarin. Als Lactid können beispielsweise Glycolid (1,4-Dioxan-2,5-dion), L-Lactid (L-3,6-Dimethyl-1,4-dioxan-2,5-dion), D-Lactid, DL-Lactid, Mesolactid und 3-Methyl-1,4-dioxan-2,5-dion, 3-Hexyl-6-methyl-1,4-dioxane-2,5-dione, 3,6-Di(but-3-en-1-yl)-1,4-dioxane-2,5-dion (jeweils einschließlich optisch aktiver Formen) eingesetzt werden. Besonders bevorzugt wird L-Lactid eingesetzt.

Neben dem aus der ersten Verfahrensstufe erhaltenen Diol können zusätzlich Alkohole wie beispielsweise Ethylenglykol, Butylenglykol, Diethylenglykol, Triethylenglykol, Polyalkylenglykole wie Polyethylenglykol, weiterhin 1,2-Propandiol, 1,3-Propandiol, 1,3-Butandiol, 1,4-Butandiol, 1,5-Pentandiol, 1,6-Hexandiol und Isomere, Neopentylglykol oder Hydroxypivalinsäureneopentylglykolester, Polyole wie Trimethylolpropan, Glycerin, Erythrit, Pentaerythrit, Trimethylolbenzol oder Trishydroxyethylisocyanurat. Vorzugsweise verwendet werden Ethylenglykol, Diethylenglykol, 1,4-Butandiol, 1,5-Pentandiol, 1,6-Hexandiol, Glycerin und Trimethylolpropan oder Mischungen aus mindestens zwei der genannten Alkohole.

Polyetheresterpolyole sind solche Verbindungen, die Ethergruppen, Estergruppen und OH-Gruppen enthalten. Organische Dicarbonsäuren mit bis zu 12 Kohlenstoffatomen sind zur Herstellung der Polyetheresterpolyole geeignet, vorzugsweise aliphatische Dicarbonsäuren mit 4 bis 6 Kohlenstoffatomen oder aromatische Dicarbonsäuren, die einzeln oder im Gemisch verwendet werden. Beispielhaft seien Korksäure, Azelainsäure, Decandicarbonsäure, Maleinsäure, Malonsäure, Phthalsäure, Pimelinsäure und Sebacinsäure sowie insbesondere Glutarsäure, Fumarsäure, Bernsteinsäure, Adipinsäure, Phthalsäure, Terephthalsäure und Isoterephthalsäure genannt. Neben organischen Dicarbonsäuren können auch cyclischen Carbonsäureanhydride, nicht-cyclische Ester und/oder cyclische Ester eingesetzt werden. Der anteilige Einsatz der oben genannten biobasierten Ausgangsstoffe, insbesondere von Fettsäuren bzw. Fettsäurederivaten (Ölsäure, Sojaöl etc.) ist ebenfalls möglich.

Als weitere Komponente zur Herstellung der Polyetheresterpolyole werden Polyetherpolyole eingesetzt, die man durch Alkoxylieren des aus dem ersten Verfahrensschritt erhaltenen Diols und gegebenenfalls weiterer H-funktioneller Startersubstanzen wie mehrwertigen Alkoholen erhält. Neben dem aus der ersten Verfahrensstufe erhaltenen Diol können zusätzlich mehrwertige Alkohole eingesetzt werden wie beispielsweise 1,2-Ethandiol, 1,3-Propandiol, 1,2-Propandiol, 1,4-Butandiol, 1,5-Pentendiol, 1,5-Pentandiol, Neopentylglykol, 1,6- Hexandiol, 1,7-Heptandiol, 1,8-Octandiol, 1,10-Decandiol, 2-Methyl-1,3-propandiol, 2,2-Dimethyl-1,3-propandiol, 3-Methyl-1,5-pentandiol, 2-Butyl-2-ethyl-1,3-propandiol, 2-Buten-1,4-diol und 2-Butin-1,4-diol, Diethylenglykol, Triethylenglykol, Tetraethylenglykol, Dibutylenglykol, Tributylenglykol, Tetrabutylenglykol, Dihexylenglykol, Trihexylenglykol, Tetrahexylenglykol, Diethylenglykol, 1,1,1-Trimethylolpropan, Triethanolamin, Glycerin, Sorbitan und Pentaerythrit sowie auf Triolen oder Tetraolen gestartete Polyethylenoxidpolyole.

Polyetheresterpolyole können auch durch die Alkoxylierung, insbesondere durch Ethoxylierung und/oder Propoxylierung, von Reaktionsprodukten, die durch die Umsetzung von organischen Dicarbonsäuren, cyclischen Carbonsäureanhydriden, nicht-cyclischen Estern und/oder cyclischen Estern sowie Komponenten mit Zerewitinoffaktiven Wasserstoffen, insbesondere Diolen und Polyolen, erhalten werden, hergestellt werden.

Herstellungsverfahren von Polyol sind beispielsweise von Ionescu in "Chemistry and Technology of Polyols for Polyurethanes", Rapra Technology Limited, Shawbury 2005, S.55 ff. (Kap. 4: Oligo-Polyols for Elastic Polyurethanes), S. 263 ff. (Kap. 8: Polyester Polyols for Elastic Polyurethanes) und insbesondere auf S.321 ff. (Kap. 13: Polyether Polyols for Rigid Polyurethane Foams) und S.419 ff. (Kap. 16: Polyester Polyols for Rigid Polyurethane Foams) beschrieben worden. Es ist auch möglich, Polyester- und Polyetherpolyole über Glykolyse von geeigneten Polymer-Rezyklaten zu gewinnen. Geeignete Polyethercarbonatpolyole und ihre Herstellung werden beispielsweise in der EP 2 910 585 A1, [0024]-[0041], beschrieben. Beispiele zu Polycarbonatpolyolen und ihre Herstellung finden sich unter anderem in der EP 1 359 177 A1. Die Herstellung geeigneter Polyetheresterpolyole ist unter anderem in der WO 2010/043624 A und in der EP 1 923 417 A beschrieben.

In einer ersten Ausführungsform betrifft die Erfindung ein Verfahren zur Herstellung von Polyol, dadurch gekennzeichnet, dass in einer ersten Verfahrensstufe ein Diol hergestellt wird durch einen Prozess enthaltend die Schritte

(1-i) Anlagerung von Alkylenoxid und Kohlendioxid an eine H-funktionelle Startersubstanz in Anwesenheit eines Katalysators, unter Erhalt von Polyethercarbonatpolyol und cyclischen Carbonats,

(1-ii) Abtrennung des cyclischen Carbonats von der resultierenden Reaktionsmischung aus Schritt (1-i),

(1-iii) Hydrolytische Spaltung des aus Schritt (1-ii) abgetrennten cyclischen Carbonats zu Kohlendioxid und Diol,

(1-iv) gegebenenfalls destillative Reinigung des aus Schritt (1-iii) resultierenden Diols.

In einer zweiten Ausführungsform betrifft die Erfindung ein Verfahren gemäß der ersten Ausführungsform, dadurch gekennzeichnet, dass in einer zweiten Verfahrensstufe

(2-i) Polyol erhalten wird durch

a) Anlagerung von Alkylenoxid und gegebenenfalls Kohlendioxid, cyclisches Carbonsäureanhydrid und/oder cyclische Ester an das in der ersten Verfahrensstufe resultierende Diol und gegebenenfalls weiterer H-funktioneller Startersubstanz

oder

b) Umsetzung von Carbonsäure, cyclischem Carbonsäureanhydrid, nicht-cyclischem Ester und/oder cyclischem Ester mit dem in der ersten Verfahrensstufe resultierenden Diol und gegebenenfalls weiteren Alkoholen.

In einer dritten Ausführungsform betrifft die Erfindung ein Verfahren gemäß der ersten oder zweiten Ausführungsform, dadurch gekennzeichnet, dass in Schritt (1-i) die Anlagerung in Anwesenheit eines Doppelmetallcyanid-Katalysators oder eines Metallkomplexkatalysators auf Basis der Metalle Zink und/oder Cobalt erfolgt.

In einer vierten Ausführungsform betrifft die Erfindung ein Verfahren gemäß einer der Ausführungsformen 1 bis 3, dadurch gekennzeichnet, dass in Schritt (1-i)

(α) eine Teilmenge H-funktioneller Startersubstanz und/oder ein Suspensionsmittel, das keine H-funktionelle Gruppen aufweist, in einem Reaktor gegebenenfalls gemeinsam mit Katalysator vorgelegt wird,

(β) gegebenenfalls zur Aktivierung eines DMC-Katalysators eine Teilmenge (bezogen auf die Gesamtmenge der bei der Aktivierung und Copolymerisation eingesetzten Menge an Alkylenoxid) von Alkylenoxid zu der aus Schritt (α) resultierenden Mischung zugesetzt wird, wobei diese Zugabe einer Teilmenge an Alkylenoxid gegebenenfalls in Gegenwart von $CO_2$ erfolgen kann, und wobei dann die aufgrund der folgenden exothermen chemischen Reaktion auftretende Temperaturspitze ("Hotspot") und/oder ein Druckabfall im Reaktor jeweils abgewartet wird, und wobei der Schritt (β) zur Aktivierung auch mehrfach erfolgen kann,

(γ) eine H-funktionelle Startersubstanz, Alkylenoxid und gegebenenfalls ein Suspensionsmittel, das keine H-funktionelle Gruppen aufweist, und/oder Kohlendioxid während der Reaktion in den Reaktor zudosiert werden,

(δ) gegebenenfalls das in Schritt (γ) kontinuierlich entfernte Reaktionsgemisch in einen Nachreaktor überführt wird, in dem im Wege einer Nachreaktion der Gehalt an freiem Alkylenoxid im Reaktionsgemisch reduziert wird.

In einer fünften Ausführungsform betrifft die Erfindung ein Verfahren gemäß einer der Ausführungsformen 1 bis 4, dadurch gekennzeichnet, dass in Schritt (1-ii) das cyclische Carbonat durch thermische Verfahren abgetrennt wird.

In einer sechsten Ausführungsform betrifft die Erfindung ein Verfahren gemäß der fünften Ausführungsform, dadurch gekennzeichnet, dass ein Dünnschichtverdampfer oder ein Fallfilmverdampfer in Kombination mit einer Strippingkolonne eingesetzt wird.

In einer siebten Ausführungsform betrifft die Erfindung ein Verfahren gemäß einer der Ausführungsformen 1 bis 6, dadurch gekennzeichnet, dass in Schritt (1-ii) das cyclische Carbonat ausgewählt ist aus mindestens einer Verbindung der Gruppe bestehend aus cyclisches Propylencarbonat und cyclisches Ethylencarbonat.

In einer achten Ausführungsform betrifft die Erfindung ein Verfahren gemäß einer der Ausführungsformen 1 bis 7, dadurch gekennzeichnet, dass das in Schritt (1-ii) erhaltene cyclische Carbonat vor Schritt (1-iii) destillativ gereinigt wird.

In einer neunten Ausführungsform betrifft die Erfindung ein Verfahren gemäß einer der Ausführungsformen 1 bis 8, dadurch gekennzeichnet, dass in Schritt (1-iii) als Hydrolyse-Katalysator mindestens eine Verbindung ausgewählt aus der Gruppe bestehend aus Alkalimetallhydroxiden, Erdalkalimetallhydroxiden und Hydrolasen eingesetzt wird.

In einer zehnten Ausführungsform betrifft die Erfindung ein Verfahren gemäß der neunten Ausführungsform, dadurch gekennzeichnet, dass in Schritt (1-iii) als Hydrolyse-Katalysator mindestens eine Verbindung ausgewählt aus der Gruppe der Alkalimetallhydroxide eingesetzt wird.

In einer elften Ausführungsform betrifft die Erfindung ein Verfahren gemäß einer der Ausführungsformen 1 bis 10, dadurch gekennzeichnet, dass 0,05 bis 1 Gew.-%, bezogen auf das in Schritt (1-iii) eingesetzte cyclische Carbonat, eines Hydrolyse-Katalysators eingesetzt wird.

In einer zwölften Ausführungsform betrifft die Erfindung ein Verfahren, gemäß einer der Ausführungsformen 1 bis 11, dadurch gekennzeichnet, dass Schritt (1-iii) bei einer Temperatur von mindestens 40 °C durchgeführt wird.

In einer dreizehnten Ausführungsform betrifft die Erfindung ein Verfahren gemäß einer der Ausführungsformen 1 bis 12, dadurch gekennzeichnet, dass in Schritt (1-iii) das molare Verhältnis von cyclischen Carbonat zu Wasser 1:1 bis 1:10 beträgt.

In einer vierzehnten Ausführungsform betrifft die Erfindung ein Verfahren gemäß einer der Ausführungsformen 1 bis 13, dadurch gekennzeichnet, dass ein Schritt (1-iv) durchgeführt wird.

In einer fünfzehnten Ausführungsform betrifft die Erfindung ein Verfahren gemäß der zweiten Ausführungsform, dadurch gekennzeichnet, dass das Polyol in Schritt (2-i) ausgewählt ist aus mindestens einer Verbindung aus der Gruppe bestehend aus Polyetherpolyol, Polyesterpolyol und Polyethercarbonatpolyol.

In einer sechszehnten Ausführungsform betrifft die Erfindung ein Verfahren gemäß der vierzehnten Ausführungsform, dadurch gekennzeichnet, dass Schritt (1-iv) bei einem Druck von 150 mbar oder darunter durchgeführt wird.

**Beispiele**

Die OH-Zahl (Hydroxylzahl) wurde gemäß DIN 53240-2 (November 2007) bestimmt.

Die Bestimmung der Viskosität erfolgte auf einem Physica MCR 501 Rheometer der Fa. Anton Paar. Es wurde eine Kegel-Platte-Konfiguration mit einem Abstand von 1 mm gewählt (Messsystem DCP25). Das Polyethercarbonatpolyol (0,1 g) wurde auf der Rheometerplatte aufgebracht und bei 25 °C einer Scherung von 0,01 bis 1000 1/s unterworfen und für 10 min alle 10 s die Viskosität gemessen. Angegeben ist die über alle Messpunkte gemittelte Viskosität.

Der Anteil an eingebautem $CO_2$ im resultierenden Polyethercarbonatpolyol sowie das Verhältnis von Propylencarbonat zu Polyethercarbonatpolyol wurden mittels $^1$H-NMR (Firma Bruker, DPX 400, 400 MHz; Pulsprogramm zg30, Wartezeit d1: 10s, 64 Scans) bestimmt. Die Probe wurde jeweils in deuteriertem Chloroform gelöst. Die relevanten Resonanzen im $^1$H-NMR (bezogen auf TMS = 0 ppm) sind wie folgt:
cyclisches Carbonat (welches als Nebenprodukt gebildet wurde) Resonanz bei 4,5 ppm, Carbonat, resultierend aus im Polyethercarbonatpolyol eingebautem Kohlendioxid (Resonanzen bei 5,1 bis 4,8 ppm), nicht abreagiertes PO mit Resonanz bei 2,4 ppm, Polyetherpolyol (d.h. ohne eingebautem Kohlendioxid) mit Resonanzen bei 1,2 bis 1,0 ppm.

Der Molenanteil des im Polymer eingebauten Carbonats in der Reaktionsmischung wird nach Formel (VIII) wie folgt berechnet, wobei folgende Abkürzungen verwendet werden: F(4,5) = Fläche der Resonanz bei 4,5 ppm für cyclisches Carbonat (entspricht einem H Atom)

F(5,1-4,8) = Fläche der Resonanz bei 5,1-4,8 ppm für Polyethercarbonatpolyol und einem H-Atom für cyclisches Carbonat.
F(2,4) = Fläche der Resonanz bei 2,4 ppm für freies, nicht abreagiertes PO
F(1,2-1,0) = Fläche der Resonanz bei 1,2-1,0 ppm für Polyetherpolyol

Unter Berücksichtigung der relativen Intensitäten wurde gemäß der folgenden Formel (VIII) für das polymer gebundene Carbonat ("lineares Carbonat" LC) in der Reaktionsmischung in mol% umgerechnet:

$$\mathrm{L}C = \frac{F(5{,}1-4{,}8)-F(4{,}5)}{\mathrm{F}(5{,}1-4{,}8)+\mathrm{F}(2{,}4)+0{,}33*\mathrm{F}(1{,}2-1{,}0)} * 100 \qquad \text{(VIII)}$$

Der Gewichtsanteil (in Gew.-%) polymer-gebundenen Carbonats (LC') in der Reaktionsmischung wurde nach Formel (IX) berechnet,

$$LC' = \frac{[F(5{,}1-4{,}8)-F(4{,}5)]*102}{N}*100\% \qquad \text{(IX)}$$

wobei sich der Wert für N ("Nenner" N) nach Formel (X) berechnet:

$$\mathrm{N} = [\mathrm{F}(5{,}1-4{,}8)-\mathrm{F}(4{,}5)]*102+\mathrm{F}(4{,}5)*102+\mathrm{F}(2{,}4)*58+0{,}33*\mathrm{F}(1{,}2-1{,}0)*58 \qquad \text{(X)}$$

Der Faktor 102 resultiert aus der Summe der Molmassen von $CO_2$ (Molmasse 44 g/mol) und der von Propylenoxid (Molmasse 58 g/mol), der Faktor 58 resultiert aus der Molmasse von Propylenoxid.

Der Gewichtsanteil (in Gew.-%) an cyclischem Carbonat (CC') in der Reaktionsmischung wurde nach Formel (XI) berechnet,

$$CC' = \frac{F(4{,}5)*102}{N}*100\% \qquad \text{(XI)}$$

wobei sich der Wert für N nach Formel (X) berechnet.

Um aus den Werten der Zusammensetzung der Reaktionsmischung die Zusammensetzung bezogen auf den Polymer-Anteil (bestehend aus Polyether, welcher aus Propylenoxid während der ggf. unter CO2-freien Bedingungen stattfindenden Aktivierungsschritten aufgebaut wurde, und Polyethercarbonatpolyol, aufgebaut aus Starter, Propylenoxid und Kohlendioxid während den in Gegenwart von $CO_2$ stattfindenden Aktivierungsschritten und während der Copolymerisation) zu berechnen, wurden die Nicht-Polymer-Bestandteile der Reaktionsmischung (d.h. cyclisches Propylencarbonat sowie ggf. vorhandenes, nicht umgesetztes Propylenoxid) rechnerisch eliminiert. Der Gewichtsanteil der Carbonat-Wiederholungseinheiten im Polyethercarbonatpolyol wurde in einen Gewichtsanteil Kohlendioxid mittels des Faktors F=44/(44+58) umgerechnet. Die Angabe des $CO_2$-Gehalts im Polyethercarbonatpolyol (siehe PECP-1) ist normiert auf das Polyethercarbonatpolyol-Molekül, das bei der Copolymerisation und den Aktivierungsschritten entstanden ist.

Die Menge an gebildeten cyclischen Propylencarbonat wird über die Bilanzierung der in der Reaktionsmischung vorhandenen Gesamtmenge an cyclischem Propylencarbonat bestimmt. Die Gesamtmenge an cyclischen Propylencarbonat ergibt sich aus der quantitativen Abtrennung des cyclischen Propylencarbonats aus der Reaktionsmischung mittels zweistufiger thermischer Aufarbeitung (Fallfilmverdampfer und Stickstoff-Strippkolonne).

Einsatzstoffe:

PECP-1: Polyethercarbonatpolyol mit einer Funktionalität von 2,8, einer OH-Zahl von 56 mg KOH/g und einem CO2-Gehalt von 20 Gew.-%
PET-1: Polyetherpolyol mit einer Funktionalität von 2 und einer OH-Zahl von 154,8 mg KOH/g
Glycerin: Fa. Aug. Hedinger GmbH & Co. KG
Adipinsäure: Fa. BASF SE
Phthalsäureanhydrid: Fa. BASF SE
1,1,1-Trimethyolpropan: Fa. BASF SE
Antioxidans: Irganox® 1076, Handelsprodukt der Firma BASF SE.

Als DMC-Katalysator wurde bei allen Beispielen DMC-Katalysator hergestellt gemäß Beispiel 6 in WO 01/80994 A1 eingesetzt.

**Beispiel 1: Herstellung von 1,2-Propylenglykol durch Abtrennung von cPC aus der Anlagerung von Propylenoxid und Kohlendioxid an H-funktioneller Startersubstanz und Hydrolyse des cPC**

Schritt (1-i):

($\alpha$) In einen kontinuierlich betriebenen 60 L Druckreaktor mit Gasdosierungseinrichtung und Produktaustragsrohr wurden 32,9 L PECP-1 enthaltend 200 ppm DMC-Katalysator vorgelegt.
($\gamma$) Bei einer Temperatur von 108 °C und einem Gesamtdruck von 65 bar (absolut) wurden unter Rühren (11 Hz) folgende Komponenten mit den angegebenen Dosierraten dosiert:

- Propylenoxid mit 7,0 kg/h
- Kohlendioxid mit bis zu 2,3 kg/h, so dass der Druck von 65 bar konstant gehalten wird
- Mischung aus Glycerin/Propylenglykol (85 Gew.-% / 15 Gew.-%) enthaltend 0,69 Gew.-% (bezogen auf die Mischung aus Glycerin und Propylenglykol) DMC-Katalysator (nicht aktiviert) und 146 ppm (bezogen auf die Mischung aus Glycerin, Propylenglykol und DMC-Katalysator) $H_3PO_4$ (eingesetzt in Form einer 85%-igen wässrigen Lösung) mit 0,27 kg/h.

Die Reaktionsmischung wurde über das Produktaustragsrohr kontinuierlich aus dem Druckreaktor entnommen, so dass das Reaktionsvolumen (32,9 L) konstant gehalten wurde, wobei die mittlere Verweilzeit des Reaktionsgemischs im Reaktor 200 min betrug.
($\delta$) Zur Vervollständigung der Reaktion wurde das entnommene Reaktionsgemisch in einen auf 120 °C temperierten Nachreaktor (Rohrreaktor mit einem Reaktionsvolumen von 2,0 L) überführt. Die mittlere Verweilzeit des Reaktionsgemischs im Nachreaktor betrug 12 min. Das Produkt wurde anschließend auf Atmosphärendruck entspannt und dann mit 500 ppm Antioxidans (Irganox® 1076) versetzt.
Danach wurde zur Ermittlung der Selektivität (Verhältnis cyclisches / lineares Carbonat) vom Reaktionsgemisch nach dem Nachreaktor eine Probe gezogen und mittels $^1$H-NMR-Analyse der Gehalt an cyclischem und linearem Carbonat bestimmt. Anschließend wurde das Produkt mittels eines Wärmetauschers auf eine Temperatur von 120 °C gebracht und unmittelbar darauf in einen 332 1 Kessel überführt und für eine Verweilzeit von 4 Stunden bei der Temperatur von 120 °C gehalten. Nach Abschluss der Verweilzeit wurde das Produkt mit 40 ppm Phosphorsäure (Komponente K) versetzt.

Schritt (1-ii):

Abschließend wurde das aus Schritt (1-i) erhaltene Reaktionsgemisch zur Abtrennung des cyclischen Propylencarbonats einer zweistufigen thermischen Aufarbeitung, nämlich in einer ersten Stufe mittels eines Fallfilmverdampfers gefolgt in einer zweiten Stufe von einer im Stickstoffgegenstrom betriebenen Strippkolonne unterworfen.
Der Fallfilmverdampfer wurde dabei bei einer Temperatur von 160 °C und einem Druck von 10 mbar (absolut) betrieben. Der verwendete Fallfilmverdampfer bestand aus Glas mit einer Austauschfläche von 0,5 $m^2$. Der Apparat besaß ein von außen beheiztes Rohr mit einem Durchmesser von 115 mm und ca. 1500 mm Länge.
Die Stickstoff-Strippkolonne wurde bei einer Temperatur von 160 °C, einem Druck von 80 mbar (absolut) und einer Stickstoff-Durchflussmenge von 0,6 kg $N_2$ / kg Produkt betrieben. Bei der verwendeten Strippkolonne handelte es sich um eine DN80 Glaskolonne mit 8 m Füllhöhe an Füllkörpern (Raschig Super-Ringe #0,3).

Schritt (1-iii):

In einem 10 L 4-Hals-Glaskolben, ausgestattet mit mechanischem Rührwerk, Thermometer und Intensivrückflußkühler mit nachgeschalteter Gasuhr, wurden 6630 g (65 Mol) cPC aus Schritt (1-ii) und eine Lösung aus 2340 g (130 Mol) Wasser und 23,4 g KOH vorgelegt. Die Lösung wurde unter Rühren zum Rückfluss erhitzt, wobei $CO_2$ abgespalten wurde. Nachdem die CO2-Entwicklung sich deutlich verlangsamt hat (nach der Bildung von 1030 L $CO_2$) wurden 1530 g (15 Mol) cPC aus Schritt (1-ii) zudosiert und die Reaktion unter den vorgenannten Reaktionsbedingungen so lange fortgeführt, bis die CO2-Entwicklung vollständig zum Erliegen kam.

Schritt (1-iv):

Das aus Schritt (1-iii) erhaltene und 1,2-Propylenglykol enthaltende Reaktionsgemisch wurde fraktioniert destilliert, wobei zur Wasserabtrennung eine Füllkörperkolonne (ca. 50 cm), eine Destillationsbrücke, sowie eine Vakuummembranpumpe eingesetzt wurden.
Nach vollständiger Abtrennung des Wassers wurde ohne Füllkörperkolonne über eine Destillationsbrücke 1,2-Propylenglykol abdestilliert, wobei die Temperatur des Destillationssumpfes 90 °C und die Kopftemperatur 85 °C bei einem Vakuum von 10 mbar betrugen.
Es wurden 5500 g (ca. 72 Mol) 1,2-Propylenglykol abdestilliert und 500 g Rückstand im Kolben belassen. Letzteres enthält das eingesetzte Kaliumhydroxid und kann für weitere Hydrolysereaktionen verwendet werden.

Analyse des erhaltenen 1,2-Propylenglykols:

| | |
|---|---|
| Wassergehalt: | 0,05 Gew.-% |
| Reinheit: | 99,5 % ($^1$H-NMR) |
| Hydroxylzahl: | 1460 mg KOH/g |

**Beispiel 2 (erfindungsgemäß): Verwendung des 1,2-Propylenglykols aus Beispiel 1 als H-funktionelle Startersubstanz für Polyetherpolyole**

Schritt (2-i):

a) In einem 10 L-Laborautoklaven wurden 500 g PET-1, welches bereits 180 ppm aktivierten DMC-Katalysator enthielt, vorgelegt. Der Inhalt des Autoklaven wurde dann unter Rühren (200 U/min, Gitterrührer) 30 min lang bei angelegtem Vakuum und unter gleichzeitigem Durchleiten von Stickstoff (50 ml/min) bei 130 °C gestrippt. Es stellte sich dabei ein Druck von 100 mbar ein. Kurz vor Beginn der Dosierphase wurde die Stickstoffeinleitung gestoppt und die Dosierung des Propylenoxids bei 24 mbar begonnen, sowie nach dem Ende der Strippzeit die Rührerdrehzahl auf 450 U/min erhöht. Insgesamt wurden 2325,2 g Propylenoxid und 175,7 g des 1,2-Propylenglykols aus Beispiel 1 bei 130 °C in den Reaktor dosiert. Zunächst wurden 23 g Propylenoxid in den Reaktor dosiert und mit der Paralleldosierung des 1,2-Propylenglykols wurde erst nach Erreichen dieser Menge begonnen. Die Dosierung des 1,2-Propylenglykols wurde so gestaltet, dass nach Ende der Dosierung der Gesamtmenge des 1,2-Propylenglykols noch 600 g Propylenoxid alleine in den Reaktor dosiert wurden. Das Propylenoxid wurde über einen unter der

Flüssigkeitsoberfläche befindlichen Sparger dosiert, wohingegen das 1,2-Propylenglykol in den Kopfraum des Autoklaven dosiert wurde. Die Dosierzeit des Propylenoxids betrug insgesamt 2,1 Stunden. Der am Ende der Dosierphase des 1,2-Propylenglykols erreichte Druck betrug 0,81 bar und fiel bei der sich anschließenden, ausschließlich Propylenoxid umfassenden, Dosierphase um 0,30 bar ab.
Nach Ende der Propylenoxiddosierung wurde die Reaktionsmischung im Zuge einer Nachreaktion noch 20 min bei den vorgenannten Bedingungen gehalten und anschließend bei 130 °C und einem Vakuum von 7 mbar über einen Zeitraum von 30 min ausgeheizt. Nach Abkühlen auf 80 °C wurden 400 ppm Antioxidans (Irganox 1076®) zugegeben. Die OH-Zahl des erhaltenen Produkts betrug 112,1 mg KOH / g und es wurde eine Viskosität von 158 mPas (bei 25 °C) ermittelt.

**Beispiel 3 (Vergleich): Verwendung von handelsüblichem 1,2-Propylenglykol (Fa. INEOS) als H-funktionelle Startersubstanz für Polyetherpolyole**

Schritt (2-i):

a) In einem 10 L-Laborautoklaven wurden 500 g PET-1, welches bereits 180 ppm aktivierten DMC-Katalysator enthielt, vorgelegt. Der Inhalt des Autoklaven wurde dann unter Rühren (200 U/min, Gitterrührer) 30 min lang bei angelegtem Vakuum und unter gleichzeitigem Durchleiten von Stickstoff (50 ml/min) bei 130 °C gestrippt. Es stellte sich dabei ein Druck von 100 mbar ein. Kurz vor Beginn der Dosierphase wurde die Stickstoffeinleitung gestoppt und die Dosierung des Propylenoxids bei 14 mbar begonnen, sowie nach dem Ende der Strippzeit die Rührerdrehzahl auf 450 U/min erhöht. Insgesamt wurden 2325,1 g Propylenoxid und 175,7 g des handelsüblichen Propylenglykols bei 130 °C in den Reaktor dosiert. Zunächst wurden 23 g Propylenoxid alleine in den Reaktor dosiert, mit der Paralleldosierung des Propylenglykols wurde nach erst Erreichen dieser Menge begonnen. Die Dosierung des Propylenglykols wurde so gestaltet, dass nach Ende der Dosierung der Gesamtmenge des Propylenglykols noch 600 g Propylenoxid alleine in den Reaktor dosiert wurden. Das Propylenoxid wurde über einen unter der Flüssigkeitsoberfläche befindlichen Sparger dosiert, wohingegen das Propylenglykol in den Kopfraum des Autoklaven dosiert wurde. Die Dosierzeit des Propylenoxids betrug insgesamt 2,1 Stunden. Der am Ende der Dosierphase des Propylenglykols erreichte Druck betrug 0,99 bar und fiel bei der sich anschließenden, ausschließlich Propylenoxid umfassenden, Dosierphase um 0,34 bar ab.
Nach Ende der Propylenoxiddosierung wurde die Reaktionsmischung im Zuge einer Nachreaktion noch 20 min bei den vorgenannten Bedingungen gehalten und anschließend bei 130 °C und einem Vakuum von 15 mbar über einen Zeitraum von 30 min ausgeheizt. Nach Abkühlen auf 80 °C wurden 400 ppm Antioxidans (Irganox 1076®) zugegeben. Die OH-Zahl des erhaltenen Produkts betrug 112,0 mg KOH / g und es wurde eine Viskosität von 157 mPas (bei 25 °C) ermittelt.

**Beispiel 4 (erfindungsgemäß): Verwendung des 1,2-Propylenglykols aus Beispiel 1 als Diol für Polyesterpolyole**

Schritt (2-i):

b) In einem 4 L-Mehrhalskolben, ausgestattet mit mechanischem Rührwerk, Innenthermometer, Heizpilz, Füllkörperkolonne, Destillationsbrücke und Membran-Vakuumpumpe, wurden 1239 g (8,48 Mol) Adipinsäure, 114,6 g (0,77 Mol) Phthalsäureanhydrid, 254 g (1,89 Mol) 1,1,1-Trimethyolpropan und 727 g (9,55 Mol) 1,2-Propylenglykol aus Beispiel 1 vorgelegt und unter Rühren im Verlauf von 50 Minuten auf 175 °C erhitzt, wobei Reaktionswasser abdestillierte. Die Reaktionstemperatur wurde innerhalb von 30 Minuten auf 200 °C erhöht und die Reaktion wurde unter diesen Bedingungen für weitere 2 Stunden fortgeführt. Anschließend wurde das Vakuum im Verlauf von 60 Minuten auf 100 mbar verringert und die Reaktion für weitere 16 Stunden fortgeführt. Die in der Vakuumphase abdestillierten 52,3 g (0,69 Mol) 1,2-Propylenglykol wurde zu der Reaktionsmischung ergänzt und die Reaktionsmischung bei Normaldruck für 6 Stunden bei 200 °C erhitzt.

Analyse:

| | |
|---|---|
| OH-Zahl: | 164,3 mg KOH/g |
| Säurezahl: | 0,4 mg KOH/g |
| Viskosität: | 810 mPas (75 °C) |
| | 3210 mPas (50 °C) |
| | 25600 mPas (25 °C) |
| Gesamtreaktionszeit: | 28,5 Stunden |

**Beispiel 5 (Vergleich): Verwendung von handelsüblichem 1,2-Propylenglykol (Fa. Bernd Kraft, Duisburg) als Diol für Polyesterpolyole**

Schritt (2-i):

b) In einem 4 L-Mehrhalskolben, ausgestattet mit mechanischem Rührwerk, Innenthermometer, Heizpilz, Füllkörperkolonne, Destillationsbrücke und Membran-Vakuumpumpe, wurden 1239 g (8,48 Mol) Adipinsäure, 114,6 g (0,77 Mol) Phthalsäureanhydrid, 254 g (1,89 Mol) 1,1,1-Trimethyolpropan und 727 g (9,55 Mol) 1,2-Propylenglykol (Fa. Bernd Kraft, Duisburg) vorgelegt und unter Rühren im Verlauf von 50 Minuten auf 175 °C erhitzt, wobei Reaktionswasser abdestillierte. Die Reaktionstemperatur wurde innerhalb von 30 Minuten auf 200 °C erhöht und die Reaktion wurde unter diesen Bedingungen für weitere 2 Stunden fortgeführt. Anschließend wurde das Vakuum im Verlauf von 60 Minuten auf 100 mbar verringert und die Reaktion für weitere 16 Stunden fortgeführt. Die in der Vakuumphase abdestillierten 49,7 g (0,65 Mol) 1,2-Propylenglykol (Fa. Bernd Kraft, Duisburg) wurden zu der Reaktionsmischung ergänzt und die Reaktionsmischung bei Normaldruck für 6 Stunden bei 200 °C erhitzt.

Analyse:

| | |
|---|---|
| OH-Zahl: | 165,2 mg KOH/g |
| Säurezahl: | 0,5 mg KOH/g |
| Viskosität: | 900 mPas (75 °C) |
| | 3500 mPas (50 °C) |
| | 28000 mPas (25 °C) |
| Gesamtreaktionszeit: | 28,5 Stunden |

Der Vergleich der Beispiele 2 und 4 mit den Beispielen 3 und 5 zeigt, dass das durch Hydrolyse von cyclischem Propylencarbonat erhaltene 1,2-Propylenglykol aus Beispiel 1 sich bei seiner Verwendung als Diolkomponente bei der Herstellung eines Polyetherpolyols (Beispiel 2) oder eines Polyesterpolyols (Beispiel 4) nicht von handelsüblichen 1,2-Propylenglykol unterscheidet. Die Polyole weisen keine relevanten Unterschiede bei der OH-Zahl, Säurezahl oder Viskosität auf.

## Patentansprüche

1. Verfahren zur Herstellung von Polyol, **dadurch gekennzeichnet, dass** in einer ersten Verfahrensstufe ein Diol hergestellt wird durch einen Prozess enthaltend die Schritte

   (1-i) Anlagerung von Alkylenoxid und Kohlendioxid an eine H-funktionelle Startersubstanz in Anwesenheit eines Katalysators, unter Erhalt von Polyethercarbonatpolyol und cyclischen Carbonats,
   (1-ii) Abtrennung des cyclischen Carbonats von der resultierenden Reaktionsmischung aus Schritt (1-i),
   (1-iii) Hydrolytische Spaltung des aus Schritt (1-ii) abgetrennten cyclischen Carbonats zu Kohlendioxid und Diol,
   (1-iv) gegebenenfalls destillative Reinigung des aus Schritt (1-iii) resultierenden Diols.

2. Verfahren gemäß Anspruch 1, **dadurch gekennzeichnet, dass** in einer zweiten Verfahrensstufe

(2-i) Polyol erhalten wird durch

a) Anlagerung von Alkylenoxid und gegebenenfalls Kohlendioxid, cyclisches Carbonsäureanhydrid und/oder cyclische Ester an das in der ersten Verfahrensstufe resultierende Diol und gegebenenfalls weiterer H-funktioneller Startersubstanz
oder
b) Umsetzung von Carbonsäure, cyclischem Carbonsäureanhydrid, nicht-cyclischem Ester und/oder cyclischem Ester mit dem in der ersten Verfahrensstufe resultierenden Diol und gegebenenfalls weiteren Alkoholen.

3. Verfahren gemäß Anspruch 1 oder 2, **dadurch gekennzeichnet, dass** in Schritt (1-i) die Anlagerung in Anwesenheit eines Doppelmetallcyanid-Katalysators oder eines Metallkomplexkatalysators auf Basis der Metalle Zink und/oder Cobalt erfolgt.

4. Verfahren gemäß Anspruch 1 bis 3, **dadurch gekennzeichnet, dass** in Schritt (1-i)

(α) eine Teilmenge H-funktioneller Startersubstanz und/oder ein Suspensionsmittel, das keine H-funktionelle Gruppen aufweist, in einem Reaktor gegebenenfalls gemeinsam mit Katalysator vorgelegt wird,
(β) gegebenenfalls zur Aktivierung eines DMC-Katalysators eine Teilmenge (bezogen auf die Gesamtmenge der bei der Aktivierung und Copolymerisation eingesetzten Menge an Alkylenoxid) von Alkylenoxid zu der aus Schritt (α) resultierenden Mischung zugesetzt wird, wobei diese Zugabe einer Teilmenge an Alkylenoxid gegebenenfalls in Gegenwart von $CO_2$ erfolgen kann, und wobei dann die aufgrund der folgenden exothermen chemischen Reaktion auftretende Temperaturspitze ("Hotspot") und/oder ein Druckabfall im Reaktor jeweils abgewartet wird, und wobei der Schritt (β) zur Aktivierung auch mehrfach erfolgen kann,
(γ) eine H-funktionelle Startersubstanz, Alkylenoxid und gegebenenfalls ein Suspensionsmittel, das keine H-funktionelle Gruppen aufweist, und/oder Kohlendioxid während der Reaktion in den Reaktor zudosiert werden,
(δ) gegebenenfalls das in Schritt (γ) kontinuierlich entfernte Reaktionsgemisch in einen Nachreaktor überführt wird, in dem im Wege einer Nachreaktion der Gehalt an freiem Alkylenoxid im Reaktionsgemisch reduziert wird.

5. Verfahren gemäß einem der Ansprüche 1 bis 4, **dadurch gekennzeichnet, dass** in Schritt (1-ii) das cyclische Carbonat durch thermische Verfahren abgetrennt wird.

6. Verfahren gemäß Anspruch 5, **dadurch gekennzeichnet, dass** ein Dünnschichtverdampfer oder ein Fallfilmverdampfer in Kombination mit einer Strippingkolonne eingesetzt wird.

7. Verfahren gemäß einem der Ansprüche 1 bis 6, **dadurch gekennzeichnet, dass** in Schritt (1-ii) das cyclische Carbonat ausgewählt ist aus mindestens einer Verbindung der Gruppe bestehend aus cyclisches Propylencarbonat und cyclisches Ethylencarbonat.

8. Verfahren gemäß einem der Ansprüche 1 bis 7, **dadurch gekennzeichnet, dass** das in Schritt (1-ii) erhaltene cyclische Carbonat vor Schritt (1-iii) destillativ gereinigt wird.

9. Verfahren gemäß einem der Ansprüche 1 bis 8, **dadurch gekennzeichnet, dass** in Schritt (1-iii) als Hydrolyse-Katalysator mindestens eine Verbindung ausgewählt aus der Gruppe bestehend aus Alkalimetallhydroxiden, Erdalkalimetallhydroxiden und Hydrolasen eingesetzt wird.

10. Verfahren gemäß Anspruch 9, **dadurch gekennzeichnet, dass** in Schritt (1-iii) als Hydrolyse-Katalysator mindestens eine Verbindung ausgewählt aus der Gruppe der Alkalimetallhydroxide eingesetzt wird.

11. Verfahren gemäß einem der Ansprüche 1 bis 10, **dadurch gekennzeichnet, dass** 0,05 bis 1 Gew.-%, bezogen auf das in Schritt (1-iii) eingesetzte cyclische Carbonat, eines Hydrolyse-Katalysators eingesetzt wird.

12. Verfahren, gemäß einem der Ansprüche 1 bis 11, **dadurch gekennzeichnet, dass** Schritt (1-iii) bei einer Temperatur von mindestens 40 °C durchgeführt wird.

13. Verfahren gemäß einem der Ansprüche 1 bis 12, **dadurch gekennzeichnet, dass** in Schritt (1-iii) das molare Verhältnis von cyclischen Carbonat zu Wasser 1:1 bis 1:10 beträgt.

**14.** Verfahren gemäß einem der Ansprüche 1 bis 13, **dadurch gekennzeichnet, dass** ein Schritt (1-iv) durchgeführt wird.

**15.** Verfahren gemäß Anspruch 2, **dadurch gekennzeichnet, dass** das Polyol in Schritt (2-i) ausgewählt ist aus mindestens einer Verbindung aus der Gruppe bestehend aus Polyetherpolyol, Polyesterpolyol und Polyethercarbonatpolyol.

Europäisches Patentamt
European Patent Office
Office européen des brevets

# EUROPÄISCHER RECHERCHENBERICHT

Nummer der Anmeldung
EP 18 17 9395

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (IPC) |
| A | WO 2017/220520 A1 (COVESTRO DEUTSCHLAND AG [DE]) 28. Dezember 2017 (2017-12-28)<br>* Ansprüche *<br>* Seite 3, Zeilen 1-5 *<br>----- | 1-15 | INV.<br>C08G64/34<br>C08G64/40<br>C08G65/26<br>C08G65/30 |
| A | WO 2004/056793 A1 (SHELL INT RESEARCH [NL]; VON HEBEL KLAAS LAMBERTUS [NL]; LANGE JEAN-PA) 8. Juli 2004 (2004-07-08)<br>* Ansprüche *<br>----- | 1-15 | |
| | | | RECHERCHIERTE SACHGEBIETE (IPC) |
| | | | C09J<br>C08G |

Der vorliegende Recherchenbericht wurde für alle Patentansprüche erstellt

2

| Recherchenort | Abschlußdatum der Recherche | Prüfer |
|---|---|---|
| Den Haag | 5. Dezember 2018 | Schlicke, Benedikt |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

# ANHANG ZUM EUROPÄISCHEN RECHERCHENBERICHT ÜBER DIE EUROPÄISCHE PATENTANMELDUNG NR.

EP 18 17 9395

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

05-12-2018

| Im Recherchenbericht angeführtes Patentdokument | Datum der Veröffentlichung | Mitglied(er) der Patentfamilie | Datum der Veröffentlichung |
|---|---|---|---|
| WO 2017220520 A1 | 28-12-2017 | EP 3260483 A1 | 27-12-2017 |
| | | WO 2017220520 A1 | 28-12-2017 |
| WO 2004056793 A1 | 08-07-2004 | AU 2003303225 A1 | 14-07-2004 |
| | | BR 0317473 A | 16-11-2005 |
| | | CN 1729182 A | 01-02-2006 |
| | | EP 1578739 A1 | 28-09-2005 |
| | | JP 2006514028 A | 27-04-2006 |
| | | KR 20050085768 A | 29-08-2005 |
| | | MX PA05006652 A | 16-08-2005 |
| | | TW 200503997 A | 01-02-2005 |
| | | US 2004152927 A1 | 05-08-2004 |
| | | WO 2004056793 A1 | 08-07-2004 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2015075057 A1
- WO 2015091246 A1
- WO 2016188838 A1
- WO 2016188992 A1
- WO 2009071651 A1
- US 6080897 A
- EP 1359177 A
- US 3404109 A
- US 3829505 A
- US 3941849 A
- US 5158922 A
- US 5470813 A
- EP 700949 A
- EP 743093 A
- EP 761708 A
- WO 9740086 A
- WO 9816310 A
- WO 0047649 A
- JP 4145123 B
- WO 0139883 A
- WO 0180994 A
- US 7304172 B2
- US 20120165549 A1
- EP 3164443 A1
- EP 2910585 A1
- EP 1359177 A1
- WO 2010043624 A
- EP 1923417 A
- WO 0180994 A1


### In der Beschreibung aufgeführte Nicht-Patentliteratur


- **B. INOUE et al.** Copolymerization of Carbon Dioxide and Epoxide with Organometallic Compounds. *Die Makromolekulare Chemie,* 1969, vol. 130, 210-220
- **M. H. CHISHOLM et al.** *Macromolecules,* 2002, vol. 35, 6494
- **S. D. ALLEN.** *J. Am. Chem. Soc.,* 2002, vol. 124, 14284
- **M. R. KEMBER et al.** *Angew. Chem., Int. Ed.,* 2009, vol. 48, 931
- **IONESCU.** Chemistry and Technology of Polyols for Polyurethanes. Rapra Technology Limited, 2005, 55 ff, , 263ff, , 321ff, , 419ff